(19) Europäisches Patentamt / European Patent Office / Office européen des brevets

(11) **EP 2 901 974 B1**

(12) **EUROPEAN PATENT SPECIFICATION**

(45) Date of publication and mention of the grant of the patent:
**12.12.2018 Bulletin 2018/50**

(21) Application number: **13840603.8**

(22) Date of filing: **12.09.2013**

(51) Int Cl.:
***A61F 13/15*** *(2006.01)* ***A61F 13/472*** *(2006.01)*
***A61F 13/511*** *(2006.01)* ***A61F 13/475*** *(2006.01)*
***A61F 13/513*** *(2006.01)* ***A61F 13/84*** *(2006.01)*

(86) International application number:
**PCT/JP2013/074732**

(87) International publication number:
**WO 2014/050598 (03.04.2014 Gazette 2014/14)**

# (54) NONWOVEN FABRIC AND ABSORBENT ARTICLE

VLIESSTOFF UND SAUGFÄHIGER ARTIKEL

TISSU NON TISSÉ ET ARTICLE ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.09.2012 JP 2012218742**

(43) Date of publication of application:
**05.08.2015 Bulletin 2015/32**

(73) Proprietor: **Unicharm Corporation**
**Ehime 799-0111 (JP)**

(72) Inventors:
- **NAKASHITA, Masashi**
**Kanonji-shi**
**Kagawa 769-1602 (JP)**
- **NODA, Yuki**
**Kanonji-shi**
**Kagawa 769-1602 (JP)**

(74) Representative: **Dolleymores**
**9 Rickmansworth Road**
**Watford, Hertfordshire WD18 0JU (GB)**

(56) References cited:
**WO-A2-2009/013660**
**JP-A- 2008 025 078**
**JP-A- 2010 285 735**
**JP-A- 2011 510 801**
**US-A1- 2007 298 220**
**US-A1- 2012 226 250**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 901 974 B1

## Description

### Technical Field

The present disclosure relates to a nonwoven fabric, and to an absorbent article comprising the nonwoven fabric as a top sheet.

### Background Art

As the basic performance of absorbent articles, such as sanitary napkins and panty liners has continued to improve with technological development over many years, leakage after absorption of excreta, such as menstrual blood has become a less frequent occurrence than in the past, and research is currently ongoing with the aim of achieving even higher performance, including a feel similar to underwear, and smoothness of the top sheet even after absorption of excreta, such as menstrual blood.

Menstrual blood during menstruation, in particular, can also contain components of the endometrium which are highly viscous, and the top sheet preferably remains smooth and stick-free even after absorption of such highly viscous menstrual blood. Highly viscous menstrual blood usually remains on the top sheet in the form of masses, generally leaving the user with a visually unpleasant image, and therefore from this viewpoint as well it is preferred for no highly viscous menstrual blood to remain on the top sheet.

Nonwoven fabrics for use in top sheets of absorbent articles in the relevant technical field include the one described in PTL 1, for example. The nonwoven fabric described in Reference 1 is designed for the purpose of providing a nonwoven fabric with modification of at least the fiber orientation, so as to have a prescribed strength even when subjected to line tension.

Absorbent articles are also known in the technical field that are coated with lotion compositions.

For example, PTL 2 discloses an absorbent article having a polypropylene glycol material-containing lotion composition situated on the inner surface of the top sheet (the clothing side surface), the inner surface of the back sheet (the body side surface), and on the base material between the inner surface of the top sheet and the inner surface of the back sheet.

Also, PTL 3 discloses an absorbent article wherein a polypropylene glycol material-containing lotion composition is applied on the outer surface of the top sheet (body side surface).

### Citation List

#### Patent Literature

PTL 1 Japanese Unexamined Patent Publication No. 2008-25078
PTL 2 Japanese Unexamined Patent Publication No. 2010-518918
PTL 3 Japanese Unexamined Patent Publication No. 2011-510801

### Summary of Invention

#### Technical Problem

The nonwoven fabric described in PTL 1 has regions with a high content of longitudinally oriented fibers and transversely oriented fibers, and maintains a prescribed strength even when subjected to line tension, but because it has regions with a high content of longitudinally oriented fibers, absorbed menstrual blood diffuses in the longitudinal direction when it is used as a top sheet, and the top sheet thus tends to be reddened.

It is therefore an object of the present disclosure to provide a nonwoven fabric for an absorbent article top sheet, that has low stickiness and is light after absorption of menstrual blood, and that has low diffusion of absorbed menstrual blood on the nonwoven fabric.

#### Solution to Problem

The present invention provides the nonwoven fabric of independent claim 1 for a top sheet of an absorbent article and the absorbent article of claim 13 for absorbing blood. The dependent claims specify preferred but optional features.

The invention provides a nonwoven fabric for a top sheet of an absorbent article, which has a longwise direction and a crosswise direction, wherein the nonwoven fabric has in the crosswise direction a plurality of units each comprising 4 regions: a first region, a second region adjacent to the first region, a third region adjacent to the second region and a fourth region adjacent to the third region, which are extending in the longwise direction, the second region and fourth region having contents of fibers oriented in the longwise direction that are higher than that in the third region, the first region having a content of fibers oriented in the crosswise direction that is higher than that in the third region, the second region, third region and fourth region respectively having a blood slipping agent-containing second region, a blood slipping agent-containing third region and a blood slipping agent-containing fourth region containing a blood slipping agent with a kinematic viscosity of 0.01 to 80 $mm^2/s$ at 40°C, a water holding percentage of 0.01 to 4.0 mass% and a weight-average molecular weight of less than 1,000, and the basis weight of the blood slipping agent in the blood slipping agent-containing second region and the blood slipping agent-containing fourth region being greater than the basis weight of the blood slipping agent in the blood slipping agent-containing third region.

Advantageous Effects of Invention

The nonwoven fabric for an absorbent article top sheet according to this disclosure has low stickiness and is light after absorption of menstrual blood, and also has low diffusion of absorbed menstrual blood on the nonwoven fabric.

Brief Description of Drawing

Fig. 1 is a perspective view of a nonwoven fabric according to an embodiment of the disclosure.
Fig. 2 is a diagram for explanation of the amount of blood slipping agent contained in the nonwoven fabric 5 shown in Fig. 1.
Fig. 3 is a perspective view of a nonwoven fabric according to another embodiment of the disclosure.
Fig. 4 is a diagram for explanation of the amount of blood slipping agent contained in the nonwoven fabric 5 shown in Fig. 3.
Fig. 5 is a front view of an absorbent article 21 comprising a nonwoven fabric, according to the present disclosure.
Fig. 6 is a cross-sectional view of the blood slipping agent-containing region 27 of the absorbent article 21 shown in Fig. 5, along cross-section X-X.
Fig. 7 is an electron micrograph of the skin contact surface of a top sheet in a sanitary napkin wherein the top sheet comprises tri-C2L oil fatty acid glycerides.
Fig. 8 is a pair of photomicrographs of menstrual blood containing and not containing a blood slipping agent.
Fig. 9 is a diagram illustrating a method of measuring surface tension.

Description of Embodiments

[Definitions]

Some of the terms used herein will now be defined. • "Fibers oriented in the longwise direction"
As used herein, "fibers oriented in the longwise direction" means fibers oriented in a range of >-45° and <+45° with respect to the longwise direction.

• "Fibers oriented in the crosswise direction"

As used herein, "fibers oriented in the crosswise direction" means fibers oriented in a range of >-45° and <+45° with respect to the crosswise direction, which is perpendicular to the aforementioned longwise direction.
Fibers oriented at -45° or +45° with respect to the longwise direction (i.e. fibers also oriented at -45° or +45° with respect to the crosswise direction) are not included among either fibers oriented in the longwise direction or fibers oriented in the crosswise direction.

• "Second region" and "blood slipping agent-containing second region"

As used herein, "blood slipping agent-containing second region" means the portion of the second region that contains a blood slipping agent. Thus, when the second region contains a blood slipping agent throughout its entirety, the area of the second region and the blood slipping agent-containing second region are the same.
The relationship between the third region and the blood slipping agent-containing third region, and between

the fourth region and the blood slipping agent-containing fourth region, is identical.

• "Ridge" and "furrow"

As used herein, "ridge" means a section extending in a fixed direction higher than the other regions and "furrow" means a section extending basically in one direction lower than the other regions, with "ridges" and "furrows" being disposed alternately in the direction perpendicular to the aforementioned fixed direction.

As used herein, the "ridges" and "furrows" are distinguished by their basis weights, for convenience.

The ridges are sections having a higher basis weight than the average basis weight of the nonwoven fabric as a whole, and the furrows are have a lower basis weight than the average basis weight of the nonwoven fabric as a whole.

• "Excretory opening contact region"

As used herein, "excretory opening contact region" as it relates to the top sheet means the region of the top sheet that contacts with the excretory opening (labia minora, etc.) of the wearer. The excretory opening contact region will have a different location depending on the size of the absorbent article, and for an absorbent article with side flaps, the excretory opening contact region will usually be the inner side of the region defined by emboss disposed in a continuous or discontinuous manner surrounding a lengthwise line running through the widthwise center of the absorbent article, and the intersection with a widthwise line running through the lengthwise centers of both wing sections. Also, in the case of an absorbent article without side flaps, usually the excretory opening contact region is defined by emboss that is disposed continuously or discontinuously surrounding the widthwise center section and the lengthwise center section of the absorbent article.

• "Blood slipping agent-containing region"

As used herein, the "blood slipping agent-containing region" as it relates to the top sheet means the region of the top sheet containing the blood slipping agent. The top sheet preferably has the blood slipping agent-containing region within the excretory opening contact region, and for example, the top sheet may have the blood slipping agent-containing region in all or a portion of the excretory opening contact region. The top sheet may also have the blood slipping agent-containing region in regions other than the excretory opening contact region, for example.

The nonwoven fabric for an absorbent article top sheet according to the present disclosure will now be described in detail.

The term "nonwoven fabric for an absorbent article top sheet" may also be referred to hereunder simply as "nonwoven fabric".

Fig. 1 is a perspective view of a nonwoven fabric according to an embodiment of the disclosure. The nonwoven fabric 5 shown in Fig. 1 has a longwise direction L and a crosswise direction C. The nonwoven fabric 5 shown in Fig. 1 has, in the crosswise direction C, a plurality of units 6 each comprising four regions: a first region 1, a second region 2 adjacent to the first region 1, a third region 3 adjacent to the second region 2 and a fourth region 4 adjacent to the third region 3, which extend in the longwise direction L. In the nonwoven fabric 5 shown in Fig. 1, the first region 1, second region 2, third region 3 and fourth region 4 extend in the longwise direction L.

Incidentally, in the nonwoven fabric of the present disclosure, the units each comprising a first region, second region, third region and fourth region may be disposed at a fixed spacing in the crosswise direction (that is, the fourth region of one unit may have a fixed spacing between it and the first region of the next unit in the crosswise direction), or they may be disposed in a continuous manner in the crosswise direction (that is, the fourth region of one unit may be adjacent to the first region of the next unit).

Also, in the nonwoven fabric 5 shown in Fig. 1, the second region 2 and fourth region 4 have a content of fibers oriented in the longwise direction L that is higher than in the third region 3, and the first region 1 has a content of fibers oriented in the crosswise direction C that is higher than in the third region 3.

As used herein, "fibers oriented in the longwise direction" and "fibers oriented in the crosswise direction" may be shortened to "longwisely oriented fibers" and "crosswisely oriented fibers", respectively.

The nonwoven fabric 5 shown in Fig. 1 has a plurality of ridges 7 and a plurality of furrows 8 extending in the longwise direction L, the first regions 1 being furrows 8 and the second regions 2, third regions 3 and fourth regions 4 being ridges 7, and more specifically, each third region 3 being the center section 7'' of a ridge and the second region 2 and fourth region 4 being the sides 7' of the ridge.

In the nonwoven fabric 5 shown in Fig. 1, the basis weight of the ridges 7 is greater than the basis weight of the furrows 8.

Fig. 2 is a diagram for explanation of the amount of blood slipping agent contained in the nonwoven fabric 5 shown in Fig. 1. In the nonwoven fabric 5 shown in Fig. 2, the second region 2, third region 3 and fourth region 4

respectively have a blood slipping agent-containing second region 12, a blood slipping agent-containing third region 13 and a blood slipping agent-containing fourth region 14, comprising a blood slipping agent with a kinematic viscosity of 0.01 to 80 $mm^2/s$ at 40°C, a water holding percentage of 0.01 to 4.0 mass% and a weight-average molecular weight of less than 1,000, the basis weight of the blood slipping agent in the blood slipping agent-containing second region 12 and the blood slipping agent-containing fourth region 14 being higher than the basis weight of the blood slipping agent in the blood slipping agent-containing third region 13.

Fig. 3 is a perspective view of a nonwoven fabric according to another embodiment of the disclosure. The nonwoven fabric 5 shown in Fig. 3 has a longwise direction L and a crosswise direction C, and has a plurality of units 6 in the crosswise direction C, each comprising four regions: a first region 1 extending in the longwise direction L, a second region 2 adjacent to the first region 1, a third region 3 adjacent to the second region 2 and a fourth region 4 adjacent to the third region 3. In the nonwoven fabric 5 shown in Fig. 3, the first region 1, second region 2, third region 3 and fourth region 4 extend in the longwise direction L.

Also, in the nonwoven fabric 5 shown in Fig. 3, the second region 2 and fourth region 4 have a content of fibers oriented in the longwise direction L that is higher than in the third region 3, and the first region 1 has a content of fibers oriented in the crosswise direction C that is higher than in the third region 3.

Fig. 4 is a diagram for explanation of the amount of blood slipping agent contained in the nonwoven fabric 5 shown in Fig. 3.

In the nonwoven fabric 5 shown in Fig. 4, the second region 2, third region 3 and fourth region 4 respectively have a blood slipping agent-containing second region 12, a blood slipping agent-containing third region 13 and a blood slipping agent-containing fourth region 14, comprising a blood slipping agent, the basis weight of the blood slipping agent in the blood slipping agent-containing second region 12 and the blood slipping agent-containing fourth region 14 being higher than the basis weight of the blood slipping agent in the blood slipping agent-containing third region 13.

In the nonwoven fabric of this disclosure, the third region has a content of longwisely oriented fibers that is preferably about 40% to about 80%. Also in the nonwoven fabric of this disclosure, the first region has a content of longwisely oriented fibers that is preferably about 0% to about 45%. In the nonwoven fabric of this disclosure, the first region has a content of longwisely oriented fibers that is preferably at least about 10% lower than the third region.

If the content of longwisely oriented fibers in the first region is lower than in the third region, i.e. if the content of crosswisely oriented fibers is higher, absorbed menstrual blood in the first region will not easily diffuse in the longwise direction and will tend to migrate into the absorbent body.

In the nonwoven fabric of this disclosure, the second region and fourth region each have a content of longwisely oriented fibers that is preferably about 55% or greater. Also in the nonwoven fabric of this disclosure, the second region and fourth region each have a content of longwisely oriented fibers that is preferably at least about 10% greater than the third region.

If the second region and fourth region have higher contents of longwisely oriented fibers than the third region, the distances between fibers will be shortened in the second region and fourth region, increasing the fiber density, and thus increasing the stiffness. As a result, the nonwoven fabric of this disclosure will be more resistant to collapse by external pressure and the like.

In the nonwoven fabric of this disclosure, the first region has a content of crosswisely oriented fibers that is preferably 55% or greater.

As used herein, the contents of longwisely oriented fibers and crosswisely oriented fibers are those measured in the following manner.

(1) A digital microscope is prepared. The digital microscope may be, for example, a VHX-100 Digital Microscope by Keyence Corp.
(2) The sample to be measured is set on the observation stage so that the longwise direction and crosswise direction are clearly identifiable.
(3) Fibers irregularly protruding forward are removed from the sample to be measured, and the lens is focused on the foremost fibers of the sample.
(4) The photographing depth is set and a 3D image of the sample is displayed on a PC screen.
(5) The photographed 3D image is converted to a 2D image.
(6) Within the measuring range, multiple parallel lines are drawn on the screen in the longwise direction and the crosswise direction, dividing it into equal portions.
(7) In each cell formed by the parallel lines, measurement is made of the number of fibers oriented in the longwise direction, the number of fibers oriented in the crosswise direction, and the number of fibers not oriented in either direction.
(8) The content of longwisely oriented fibers and the content of crosswisely oriented fibers are calculated from the total number of fibers in the prescribed range.

In a nonwoven fabric having ridges and furrows according to one embodiment of this disclosure, the ridges have a basis weight of preferably about 15 to about 250 g/m$^2$ and more preferably about 20 to about 120 g/m$^2$. If the basis weight is less than about 15 g/m$^2$, the ridges will tend to be highly collapsable, and absorbed menstrual blood will tend to rewet when pressure is applied. If the basis weight is greater than about 250 g/m$^2$, menstrual blood will not easily migrate downward (into the absorbent body, for example), and the menstrual blood will pool in the ridges, potentially creating a feeling of discomfort for the wearer.

In a nonwoven fabric having ridges and furrows according to one embodiment of this disclosure, the ridges have a basis weight of preferably about 3 to about 150 g/m$^2$ and more preferably about 5 to 80 g/m$^2$. If the basis weight is less than about 3 g/m$^2$, the formed top sheet may tear during use. Also, if the basis weight is greater than about 150 g/m$^2$, menstrual blood that has reached the furrows will not easily migrate downward (into the absorbent body), and the menstrual blood will pool in the furrows, potentially creating a feeling of discomfort for the wearer.

A nonwoven fabric having ridges and furrows according to one embodiment of this disclosure has an average basis weight of preferably about 10 to 200 g/m$^2$ and more preferably about 20 to about 100 g/m$^2$. If the average basis weight is less than about 10 g/m$^2$, the formed top sheet may tear during use. Also, if the average basis weight is greater than about 200 g/m$^2$, menstrual blood can potentially pool in the nonwoven fabric.

The basis weights of the ridges and the furrows and the average basis weight of the nonwoven fabric are measured in the following manner.

(1) A mark is created in the region to be measured (ridge, furrow or nonwoven fabric), and the area: $SA_\alpha(m^2)$ is measured.
In order to minimize error, marking is made so that the total area of the sample exceeds 5 cm$^2$.
(2) The marked area is cut with a sharp blade, for example a cutter replacement blade, and the total mass measured as TM (g).
(3) The basis weight $BS_\alpha(g/m^2)$ of the area to be measured is determined by the following formula:

$$BS_\alpha(g/m^2) = TM\ (g)/SA_\alpha(m^2).$$

In the nonwoven fabric of this disclosure, the blood slipping agent-containing second region of the second region and/or the blood slipping agent-containing fourth region of the fourth region contain the blood slipping agent at a basis weight in the range of preferably about 1 to about 30 g/m$^2$, more preferably about 2 to about 20 g/m$^2$ and even more preferably about 3 to about 10 g/m$^2$. The action of the blood slipping agent will be described below, but if the basis weight is lower than about 1 g/m$^2$, menstrual blood that has reached the nonwoven fabric will tend to remain there without rapidly migrating into the absorbent body, while if the basis weight is greater than about 30 g/m$^2$, a greater degree of stickiness will tend to be felt when the article is worn.

Also, if the second region and/or fourth region contain the blood slipping agent within this range, menstrual blood that has reached the second region and/or fourth region will tend to slip down into the absorbent article before diffusing in the longwise direction along the longwisely oriented fibers. This reduces reddening of the top sheet with menstrual blood, minimizes rash caused by menstrual blood adhering to the skin, and/or reduces repulsive appearance.

For the nonwoven fabric of this disclosure, the blood slipping agent-containing third region of the third region contains the blood slipping agent at a basis weight that is in a proportion of preferably about 1 to about 70 mass%, more preferably about 3 to about 60 mass% and even more preferably about 5 to about 50 mass% of the basis weight of the blood slipping agent in the blood slipping agent-containing second region and/or the blood slipping agent in the blood slipping agent-containing fourth region.

The action of the blood slipping agent will be explained below, but if this proportion is less than about 1 mass%, menstrual blood reaching the top sheet will tend to remain in that location without rapidly migrating into the absorbent body, and if the proportion is greater than about 70 mass%, the feeling of stickiness during wearing will tend to be increased.

Furthermore, since the third region has a low content of longwisely oriented fibers, menstrual blood that has reached the third region tends to undergo less diffusion in the longwise direction. Consequently, even if the amount of blood slipping agent in the blood slipping agent-containing third region is reduced to some extent, menstrual blood reaching the third region does not easily diffuse in the longwise direction. This amount is therefore useful from the viewpoint of cost.

In the nonwoven fabric of this disclosure, the first region may have a blood slipping agent-containing first region that contains a blood slipping agent. If the first region has a blood slipping agent-containing first region, absorbed menstrual blood will easily slip into the absorbent article before diffusing in the crosswise direction. This will minimize reddening of the top sheet by menstrual blood.

For the purpose of the present specification, the basis weight of the blood slipping agent in the nonwoven fabric is that measured in the following manner.

(1) The region of the nonwoven fabric that is to be measured is cut out using a sharp blade, such as a cutter replacement blade, avoiding any alteration in thickness, to obtain a sample.
(2) The area of the sample: $SA_\beta(m^2)$ and the mass: $SM_0$ (g) are measured.
(3) The sample is stirred for at least 3 minutes in a solvent that can dissolve the blood slipping agent, such as ethanol or acetone, to dissolve the blood slipping agent in the solvent.
(4) The sample is filtered on mass-measured filter paper, and the sample is thoroughly rinsed with the solvent on the filter paper. The sample on the filter paper is dried in an oven at 60°C.
(5) The masses of the filter paper and sample are measured, and the mass of the filter paper is subtracted to calculate the dry sample mass: $SM_1$ (g).
(6) The basis weight $BS_\beta$ ($g/m^2$) of the blood slipping agent is calculated by the following formula.

$$BS_\beta\ (g/m^2) = [SM_0\ (g) - SM_1\ (g)]/SA_\beta(m^2).$$

In order to minimize error, multiple samples are taken from multiple absorbent articles, without the total area of the sample exceeding 100 $cm^2$, conducting several repeated measurements and taking the average value.
The nonwoven fabric according to one embodiment of this disclosure contains the same blood slipping agent, or the same combination of blood slipping agents, in the blood slipping agent-containing second region of the second region, the blood slipping agent-containing third region of the third region and the blood slipping agent-containing fourth region of the fourth region.
Also, the nonwoven fabric according to another embodiment of this disclosure contains a different blood slipping agent, or a different combination of blood slipping agents, in the blood slipping agent-containing second region of the second region, the blood slipping agent-containing third region of the third region and the blood slipping agent-containing fourth region of the fourth region.

[Blood slipping agent]

In the nonwoven fabric of this disclosure, the second region, third region and fourth region have, respectively, a blood slipping agent-containing second region, a blood slipping agent-containing third region and a blood slipping agent-containing fourth region containing a blood slipping agent having a kinematic viscosity of about 0.01 to about 80 $mm^2/s$ at 40°C, a water holding percentage of about 0.05 to about 4.0 mass% and a weight-average molecular weight of less than about 1,000.
The blood slipping agent has, at 40°C, a kinematic viscosity of about 0 to about 80 $mm^2/s$, preferably a kinematic viscosity of about 1 to about 70 $mm^2/s$, more preferably a kinematic viscosity of about 3 to about 60 $mm^2/s$, even more preferably a kinematic viscosity of about 5 to about 50 $mm^2/s$, and yet more preferably a kinematic viscosity of about 7 to about 45 $mm^2/s$.
The kinematic viscosity tends to be higher with a) a larger molecular weight of the blood slipping agent, b) a higher percentage of polar groups, such as carbonyl bonds (-CO-), ether bonds (-O-), carboxyl groups (-COOH) and hydroxyl groups (-OH), and c) a larger IOB.
In order to have a kinematic viscosity of about 0 to about 80 $mm^2/s$ at 40°C, the melting point of the blood slipping agent is preferably 45°C or less. This is because the kinematic viscosity will tend to be higher if the blood slipping agent contains crystals at 40°C.
As used herein, the "kinematic viscosity at 40°C" may be referred to simply as "kinematic viscosity".
The significance of the kinematic viscosity of the blood slipping agent will be explained below, but a kinematic viscosity exceeding about 80 $mm^2/s$ will tend to result in high viscosity of the blood slipping agent, so that the blood slipping agent will tend to be resistant to slipping into the absorbent article together with menstrual blood that has reached the skin contact surface of the top sheet.
The kinematic viscosity can be measured according to JIS K 2283:2000, "5. Kinematic Viscosity Test Method", using a Cannon-Fenske reverse-flow viscometer, at a testing temperature of 40°C.
The blood slipping agent has a water holding percentage of about 0.01 to about 4.0 mass%, preferably it has a water holding percentage of about 0.02 to about 3.5 mass%, more preferably it has a water holding percentage of about 0.03 to about 3.0 mass%, even more preferably it has a water holding percentage of about 0.04 to about 2.5 mass%, and yet more preferably it has a water holding percentage of about 0.05 to about 2.0 mass%.
As used herein, "water holding percentage" means the percentage (mass) of water that can be held by a

substance, and it may be measured in the following manner.

(1) A 20 mL test tube, a rubber stopper, the substance to be measured and deionized water are allowed to stand for a day and a night in a thermostatic chamber at 40°C.
(2) Into the test tube in the thermostatic chamber there are charged 5.0 g of the substance to be measured and 5.0 g of deionized water.
(3) The mouth of the test tube is sealed with the rubber stopper in the thermostatic chamber, and the test tube is rotated once and allowed to stand for 5 minutes.
(4) A 3.0 g portion of the layer of the substance to be measured (usually the upper layer) is sampled into a glass dish with a diameter of 90 mm and a mass of $W_0$ (g), in the thermostatic chamber.
(5) The dish is heated at 105°C for 3 hours in an oven to evaporate off the moisture, and the mass $W_1$ (g) of each dish is measured.
(6) The water holding percentage is calculated by the following formula.

$$\text{Water holding percentage (mass\%)} = 100 \times [W_0\ (g) - W_1\ (g)]/3.0\ (g)$$

The measurement is conducted three times, and the average value is recorded.
The significance of the water holding percentage of the blood slipping agent will be explained below, but a low water holding percentage will tend to lower the affinity between the blood slipping agent and menstrual blood, thus helping to prevent menstrual blood that has reached the skin contact surface of the top sheet from slipping into the absorbent article.
If the water holding percentage is high, on the other hand, affinity with menstrual blood will be very high, similar to a surfactant, and absorbed menstrual blood will tend to remain on the skin contact surface of the top sheet, resulting in more red coloration of the skin contact surface of the top sheet.
The water holding percentage tends to be a larger value with a) a smaller molecular weight of the blood slipping agent, and b) a higher percentage of polar groups, such as carbonyl bonds (-CO-), ether bonds (-O-), carboxyl groups (-COOH) and hydroxyl groups (-OH). This is because the blood slipping agent has greater hydrophilicity. The water holding percentage will tend to have a larger value with a greater IOB, i.e with a higher inorganic value or with a lower organic value. This is because the blood slipping agent will have greater hydrophilicity.
The significance of the kinematic viscosity and water holding percentage of the blood slipping agent will now be explained.
Fig. 5 is a front view of an absorbent article, and more specifically a sanitary napkin, containing a nonwoven fabric of this disclosure. Fig. 5 is as observed from the skin contact side of the top sheet 22. The absorbent article 21 shown in Fig. 5 has a liquid-permeable top sheet 22, an absorbent body 23, and a liquid-impermeable back sheet (not shown). The absorbent article 21 shown in Fig. 5 also has a pair of side flaps 24, a side sheet 25 and embosses 26.
In the absorbent article 21 shown in Fig. 5, the left side is the front.
In the absorbent article 21 shown in Fig. 5, the top sheet 22 has a plurality of ridges and a plurality of furrows on the skin contact surface, extending in the longitudinal direction of the absorbent article, and the ridges and furrows may be omitted as appropriate. In the absorbent article 21 shown in Fig. 5, the ridges and furrows are disposed in an alternating fashion in the widthwise direction of the absorbent article 21.
Also, although the absorbent article 21 shown in Fig. 5 has a pair of side flaps 24, a side sheet 25 and embosses 26, an absorbent article according to another embodiment of this disclosure does not have a pair of side flaps, a side sheet and/or emboss.
In the absorbent article 21 shown in Fig. 5, the excretory opening contact region is the region defined by four embosses 26', and the top sheet 22 has a blood slipping agent-containing region 27 over the entire excretory opening contact region.
Fig. 6 is a cross-sectional view corresponding to cross-section X-X of the blood slipping agent-containing region 27 of the absorbent article 21 shown in Fig. 5, and it is a diagram schematically illustrating migration of menstrual blood into the absorbent body by the blood slipping agent. The absorbent article 21 shown in Fig. 6 has a liquid-permeable top sheet 22, a liquid-impermeable back sheet 28, and an absorbent body 23 between the top sheet 22 and the back sheet 28.
In Fig. 6, the top sheet 22 has a plurality of projections 31 and a plurality of recesses 32 on the skin contact surface 33, and a blood slipping agent 34 is coated on the skin contact surface 33 of the top sheet 22. In Fig. 6, the blood slipping agent 34 is shown as droplets (or particles) on the skin contact surface 33 of the top sheet 22 for convenience, but in a nonwoven fabric of this disclosure, and an absorbent article comprising the nonwoven fabric, the form and distribution of the blood slipping agent is not limited to that shown in the drawing.

As shown in Fig. 6, menstrual blood 35 that has reached the projections 31 of the top sheet 22 contacts with the blood slipping agent 34 that is present in the projections 31. A portion of the blood slipping agent 34 present in the projections 31 slips down into the recesses 32 together with the menstrual blood 35 (menstrual blood 35'). The menstrual blood 35' then slips down into the recesses 32, reaching the absorbent body 23 (menstrual blood 35''). Next, the menstrual blood 35'' is absorbed into the absorbent body 23.

More specifically, the blood slipping agent 34 having a water holding percentage of about 0.01 to about 4.0 mass% has a certain affinity with menstrual blood 35. For example, the hydrophilic portion of the blood slipping agent 34 (for example, a hydrophilic group, such as a polar group, for example, such as carbonyl, oxy, carboxyl, hydroxyl or the like, or a hydrophilic bond, such as a polar bond, for example, such as a carbonyl bond, ester bond, carbonate bond, ether bond or the like) has high affinity with the hydrophilic components (such as blood plasma) in the menstrual blood 35, and attracts the components with affinity, whereas the hydrophobic portion (for example, the hydrocarbon moiety) of the blood slipping agent 34 has low affinity with the hydrophilic components (such as blood plasma) in the menstrual blood 35 and repels the hydrophilic components, such that it functions as a "lubricant", causing the menstrual blood 35 to slip down toward the absorbent body 23.

Also, since the blood slipping agent 34 having a kinematic viscosity of about 0.01 to about 80 $mm^2/s$ at 40°C has very low viscosity near the body temperature of the wearer, a portion thereof slips down from the projections 31 into the recesses 32 together with the menstrual blood 35, subsequently passing through the recesses 32 into the absorbent article 21.

Furthermore, since the blood slipping agent 34 has a water holding percentage of about 0.01 to about 4.0 mass%, its affinity with the hydrophilic components (such as blood plasma) in menstrual blood 35 is not excessively high, and this causes less of the menstrual blood 35 to remain on the top sheet 22. This is because the hydrophilic components (such as blood plasma) in the menstrual blood 35 repels the hydrophobic portion of the blood slipping agent 34.

Fig. 6 schematically illustrates migration of menstrual blood into an absorbent body by a blood slipping agent, for a top sheet 22 having a plurality of projections 31 and a plurality of recesses 32 on the skin contact surface 33, but menstrual blood also migrates in the same manner in a top sheet without irregularities, such as a flat nonwoven fabric. This is because menstrual blood slips down between the fibers of the nonwoven fabric.

Although Fig. 6 schematically illustrates migration of menstrual blood into the absorbent body by a blood slipping agent, a blood slipping agent-containing composition functions in the same manner.

The blood slipping agent has a weight-average molecular weight of less than about 1,000, and preferably a weight-average molecular weight of less than about 900. This is because, if the weight-average molecular weight is about 1,000 or higher, tack may result in the blood slipping agent itself, tending to create a feeling of unpleasantness for the wearer. If the weight-average molecular weight increases, the viscosity of the blood slipping agent will tend to increase, and it will therefore be difficult to lower the viscosity of the blood slipping agent by heating to a viscosity suitable for coating, and as a result, the blood slipping agent may need to be diluted with a solvent.

The blood slipping agent preferably has a weight-average molecular weight of about 100 or greater, and more preferably it has a weight-average molecular weight of about 200 or greater. This is because if the weight-average molecular weight is low, the vapor pressure of the blood slipping agent may be increased, gasification may occur during storage and the amount may be reduced, often leading to problems, such as odor during wear.

In addition, as used herein, "weight-average molecular weight" includes the concept of a polydisperse compound (for example, a compound produced by stepwise polymerization, an ester formed from a plurality of fatty acids and a plurality of aliphatic monohydric alcohols), and a simple compound (for example, an ester formed from one fatty acid and one aliphatic monohydric alcohol), and in a system comprising $N_i$ molecules with molecular weight $M_i$ (i = 1, or i = 1, 2 ...), it refers to $M_w$ determined by the following formula.

$$M_w = \Sigma N_i M_i^2 / \Sigma N_i M_i$$

As used herein, the weight-average molecular weights are the values measured by gel permeation chromatography (GPC), based on polystyrene.

The GPC measuring conditions may be the following, for example.

Device: Lachrom Elite high-speed liquid chromatogram by Hitachi High-Technologies Corp.
Columns: SHODEX KF-801, KF-803 and KF-804, by Showa Denko K.K.
Eluent: THF
Flow rate: 1.0 mL/min
Driving volume: 100 μL

Detection: RI (differential refractometer)

The weight-average molecular weights listed in the examples of the present specification were measured under the conditions described below.

The blood slipping agent can have an IOB of about 0.00 to about 0.60.

The IOB (Inorganic Organic Balance) is an indicator of the hydrophilic-lipophilic balance, and as used herein, it is the value calculated by the following formula by Oda et al.:

$$\text{IOB} = \text{inorganic value/organic value.}$$

The inorganic value and the organic value are based on the organic paradigm described in "Organic compound predictions and organic paradigms" by Fujita A., Kagaku no Ryoiki (Journal of Japanese Chemistry), Vol.11, No.10 (1957) p.719-725.

The organic values and inorganic values of major groups, according to Fujita, are summarized in Table 1 below.

Table 1

| Group | Inorganic value | Organic value |
|---|---|---|
| -COOH | 150 | 0 |
| -OH | 100 | 0 |
| -O-CO-O- | 80 | 0 |
| -CO- | 65 | 0 |
| -COOR | 60 | 0 |
| -O- | 20 | 0 |
| Triple bond | 3 | 0 |
| Double bond | 2 | 0 |
| $CH_2$ | 0 | 20 |
| *iso*-branch | 0 | -10 |
| *tert*-branch | 0 | -20 |
| Light metal (salt) | ≥500 | 0 |
| Heavy metal (salt), amine, $NH_3$ salt | ≥400 | 0 |

For example, in the case of an ester of tetradecanoic acid which has 14 carbon atoms and dodecyl alcohol which has 12 carbon atoms, the organic value is 520 ($CH_2$, 20 × 26) and the inorganic value is 60 (-COOR, 60 × 1), and therefore IOB = 0.12.

The IOB of the blood slipping agent is preferably between about 0.00 and 0.60, more preferably between about 0.00 and 0.50, even more preferably between about 0.00 and 0.40 and most preferably between about 0.00 and 0.30. If the IOB is within this range, it will be easier to meet the aforementioned conditions for the water-holding capacity and kinematic viscosity.

The blood slipping agent preferably has a melting point of no higher than 45°C, and more preferably it has a melting point of no higher than 40°C. If the blood slipping agent has a melting point of no higher than 45°C, the blood slipping agent will more easily exhibit a kinematic viscosity in the aforementioned range.

As used herein, the term "melting point" refers to the peak top temperature for the endothermic peak during conversion from solid to liquid, upon measurement with a differential scanning calorimetry analyzer at a temperature-elevating rate of 10°C/min. The differential scanning calorimetry analyzer used may be, for example, a DSC-60-type DSC measuring apparatus by Shimadzu Corp.

If the blood slipping agent has a melting point of about 45°C or less, it may be either liquid or solid at room temperature (about 25°C), or in other words, the melting point may be either about 25°C or higher or below about 25°C, and for example, it may have a melting point of about -5°C or about -20°C. The reason for a melting point of about 45°C or less for the blood slipping agent will be explained below.

The blood slipping agent does not have a lower limit for the melting point, but the vapor pressure is preferably

low. The vapor pressure of the blood slipping agent is preferably about 0-200 Pa, more preferably about 0-100 Pa, even more preferably about 0-10 Pa, even more preferably about 0-1 Pa, and even more preferably about 0.0-0.1 Pa at 25°C (1 atmosphere).

Considering that the absorbent article of this disclosure is to be used in contact with the human body, the vapor pressure is preferably about 0-700 Pa, more preferably about 0-100 Pa, even more preferably about 0-10 Pa, even more preferably about 0-1 Pa, and even more preferably 0.0-0.1 Pa, at 40°C (1 atmosphere). If the vapor pressure is high, gasification may occur during storage and the amount of blood slipping agent may be reduced, and as a consequence problems, such as odor during wear, may be created.

The melting point of the blood slipping agent may be selected depending on the weather or duration of wear. For example, in regions with a mean atmospheric temperature of about 10°C or less, using a blood slipping agent with a melting point of about 10°C or less may help the blood slipping agent function after excretion of menstrual blood, even if it has been cooled by the ambient temperature.

Also, when the absorbent article is to be used for a prolonged period of time, the melting point of the blood slipping agent is preferably at the high end of the range of about 45°C or less. This is so that the blood slipping agent will not be easily affected by sweat or friction during wearing, and will not easily become biased even during prolonged wearing.

In the technical field, the skin contact surfaces of top sheets are coated with surfactants in order to alter the surface tension of menstrual blood and promote rapid absorption of menstrual blood. However, the top sheet coated with the surfactant has very high affinity for the hydrophilic components (blood plasma, etc.) in menstrual blood, and acts to attract them, tending to cause menstrual blood instead to remain on the top sheet. The blood slipping agent, unlike conventionally known surfactants, has low affinity with menstrual blood and therefore does not cause residue of menstrual blood on the top sheet and allows rapid migration into the absorbent body.

Preferably, the blood slipping agent is selected from the group consisting of following items (i)-(iii), and any combination thereof:

(i) a hydrocarbon;
(ii) a compound having (ii-1) a hydrocarbon moiety, and (ii-2) one or more, same or different groups selected from the group consisting of carbonyl group (-CO-) and oxy group (-O-) inserted between a C-C single bond of the hydrocarbon moiety; and
(iii) a compound having (iii-1) a hydrocarbon moiety, (iii-2) one or more, same or different groups selected from the group consisting of carbonyl group (-CO-) and oxy group (-O-) inserted between a C-C single bond of the hydrocarbon moiety, and (iii-3) one or more, same or different groups selected from the group consisting of carboxyl group (-COOH) and hydroxyl group (-OH) substituting for a hydrogen on the hydrocarbon moiety.

As used herein, "hydrocarbon" refers to a compound composed of carbon and hydrogen, and it may be a chain hydrocarbon, such as a paraffinic hydrocarbon (containing no double bond or triple bond, also referred to as alkane), an olefin-based hydrocarbon (containing one double bond, also referred to as alkene), an acetylene-based hydrocarbon (containing one triple bond, also referred to as alkyne), or a hydrocarbon comprising two or more bonds selected from the group consisting of double bonds and triple bonds, and cyclic hydrocarbon, such as aromatic hydrocarbons and alicyclic hydrocarbons.

Preferred as such hydrocarbons are chain hydrocarbons and alicyclic hydrocarbons, with chain hydrocarbons being more preferred, paraffinic hydrocarbons, olefin-based hydrocarbons and hydrocarbons with two or more double bonds (containing no triple bond) being more preferred, and paraffinic hydrocarbons being even more preferred.

Chain hydrocarbons include linear hydrocarbons and branched hydrocarbons.

When two or more oxy groups (-O-) are inserted in the compounds of (ii) and (iii) above, the oxy groups (-O-) are not adjacent each other. Thus, compounds (ii) and (iii) do not include compounds with continuous oxy groups (i.e., peroxides).

In the compounds of (iii), compounds in which at least one hydrogen on the hydrocarbon moiety is substituted with a hydroxyl group (-OH) are preferred over compounds in which at least one hydrogen on the hydrocarbon moiety is substituted with a carboxyl group (-COOH). This is because the carboxyl groups bond with metals and the like in menstrual blood, increasing the water holding percentage of the blood slipping agent, which may sometimes exceed the prescribed range. The same is true from the viewpoint of the IOB as well. As shown in Table 1, the carboxyl groups bond with metals and the like in menstrual blood, drastically increasing the inorganic value from 150 to 400 or greater, and therefore a blood slipping agent with carboxyl groups can increase the IOB value to more than about 0.60 during use.

More preferably, the blood slipping agent is selected from the group consisting of following items (i')-(iii'), and any combination thereof:

(i') a hydrocarbon;

(ii') a compound having (ii'-1) a hydrocarbon moiety, and (ii'-2) one or more, same or different bonds selected from the group consisting of carbonyl bond (-CO-), ester bond (-COO-), carbonate bond (-OCOO-), and ether bond (-O-) inserted between a C-C single bond of the hydrocarbon moiety; and

(iii') a compound having (iii'-1) a hydrocarbon moiety, (iii'-2) one or more, same or different bonds selected from the group consisting of carbonyl bond (-CO-), ester bond (-COO-), carbonate bond (-OCOO-), and ether bond (-O-) inserted between a C-C single bond of the hydrocarbon moiety, and (iii'-3) one or more, same or different groups selected from the group consisting of carboxyl group (-COOH) and hydroxyl group (-OH) substituting for a hydrogen on the hydrocarbon moiety.

When 2 or more same or different bonds are inserted in the compound of (ii') or (iii'), i.e., when 2 or more same or different bonds selected from the group consisting carbonyl bonds (-CO-), ester bonds (-COO-), carbonate bonds (-OCOO-) and ether bonds (-O-) are inserted, the bonds are not adjacent to each other, and at least one carbon atom lies between each of the bonds.

The blood slipping agent has more preferably about 1.8 or less carbonyl bonds (-CO-), about 2 or less ester bonds (-COO-), about 1.5 or less carbonate bonds (-OCOO-), about 6 or less ether bonds (-O-), about 0.8 or less carboxyl groups (-COOH) and/or about 1.2 or less hydroxyl groups (-OH), per 10 carbon atoms in the hydrocarbon moiety.

Even more preferably, the blood slipping agent is selected from the group consisting of following items (A)-(F), and any combination thereof:

(A) an ester of (A1) a compound having a chain hydrocarbon moiety and 2-4 hydroxyl groups substituting for hydrogens on the chain hydrocarbon moiety, and (A2) a compound having a chain hydrocarbon moiety and 1 carboxyl group substituting for a hydrogen on the chain hydrocarbon moiety;

(B) an ether of (B1) a compound having a chain hydrocarbon moiety and 2-4 hydroxyl groups substituting for hydrogens on the chain hydrocarbon moiety, and (B2) a compound having a chain hydrocarbon moiety and 1 hydroxyl group substituting for a hydrogen on the chain hydrocarbon moiety;

(C) an ester of (C1) a carboxylic acid, hydroxy acid, alkoxy acid or oxoacid comprising a chain hydrocarbon moiety and 2-4 carboxyl groups substituting for hydrogens on the chain hydrocarbon moiety, and (C2) a compound having a chain hydrocarbon moiety and 1 hydroxyl group substituting for a hydrogen on the chain hydrocarbon moiety;

(D) a compound having a chain hydrocarbon moiety and one bond selected from the group consisting of ether bonds (-O-), carbonyl bonds (-CO-), ester bonds (-COO-) and carbonate bonds (-OCOO-) inserted between a C-C single bond of the chain hydrocarbon moiety;

(E) a polyoxy $C_3$-$C_6$ alkylene glycol, or ester or ether thereof; and

(F) a chain hydrocarbon.

The blood slipping agent in accordance with (A) to (F) will now be described in detail.

[(A) Ester of (A1) a compound having a chain hydrocarbon moiety and 2-4 hydroxyl groups substituting for hydrogens on the chain hydrocarbon moiety, and (A2) a compound having a chain hydrocarbon moiety and 1 carboxyl group substituting for a hydrogen on the chain hydrocarbon moiety]

In the (A) ester of (A1) a compound having a chain hydrocarbon moiety and 2-4 hydroxyl groups substituting for hydrogens on the chain hydrocarbon moiety, and (A2) a compound having a chain hydrocarbon moiety and 1 carboxyl group substituting for a hydrogen on the chain hydrocarbon moiety (hereunder also referred to as "compound (A)"), it is not necessary for all of the hydroxyl groups to be esterified so long as the kinematic viscosity, water holding percentage and weight-average molecular weight are within the aforementioned ranges.

Examples of (A1) a compound having a chain hydrocarbon moiety and 2-4 hydroxyl groups substituting for hydrogens on the chain hydrocarbon moiety (hereunder also referred to as "compound (A1)") include chain hydrocarbon tetraols, such as alkanetetraols, including pentaerythritol, chain hydrocarbon triols, such as alkanetriols, including glycerins, and chain hydrocarbon diols, such as alkanediols, including glycols.

Examples of (A2) a compound having a chain hydrocarbon moiety and 1 carboxyl group substituting for a hydrogen on the chain hydrocarbon moiety include compounds in which one hydrogen on the hydrocarbon is substituted with one carboxyl group (-COOH), such as fatty acids.

Examples for compound (A) include ($a_1$) an ester of a chain hydrocarbon tetraol and at least one fatty acid, ($a_2$) an ester of a chain hydrocarbon triol and at least one fatty acid, and ($a_3$) an ester of a chain hydrocarbon diol and at least one fatty acids.

[($a_1$) Esters of a chain hydrocarbon tetraol and at least one fatty acid]

Examples of an ester of a chain hydrocarbon tetraol and at least one fatty acid include tetraesters of pentaerythritol and fatty acids, represented by the following formula (1):

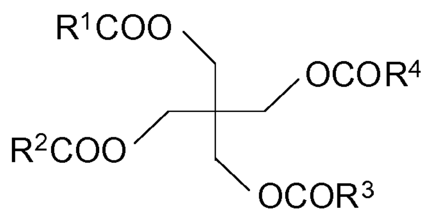

(1)

triesters of pentaerythritol and fatty acids, represented by the following formula (2):

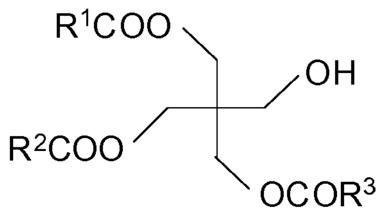

(2)

diesters of pentaerythritol and fatty acids, represented by the following formula (3):

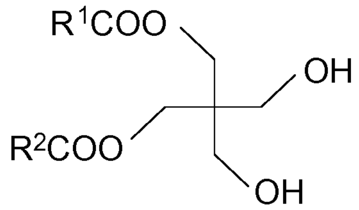

(3)

and monoesters of pentaerythritol and fatty acids, represented by the following formula (4).

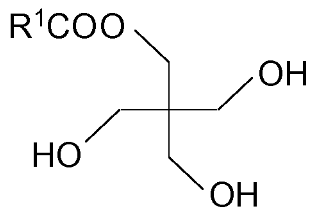

(4)

In the formulas, $R^1$-$R^4$ each represent a chain hydrocarbon.

The fatty acids consisting of the esters of pentaerythritol and fatty acids ($R^1COOH$, $R^2COOH$, $R^3COOH$, and $R^4COOH$) are not particularly restricted so long as the pentaerythritol and fatty acid esters satisfy the conditions for the kinematic viscosity, water holding percentage and weight-average molecular weight, and for example, there may be mentioned saturated fatty acids, such as a $C_2$-$C_{30}$ saturated fatty acids, including acetic acid ($C_2$) ($C_2$ representing the number of carbons, corresponding to the number of carbons of each of $R^1C$, $R^2C$, $R^3C$ or $R^4C$, same hereunder), propanoic acid ($C_3$), butanoic acid ($C_4$) and isomers thereof, such as 2-methylpropanoic acid ($C_4$), pentanoic acid ($C_5$) and isomers thereof, such as 2-methylbutanoic acid ($C_5$) and 2,2-dimethylpropanoic acid ($C_5$), hexanoic acid ($C_6$), heptanoic acid ($C_7$), octanoic acid ($C_8$) and isomers thereof, such as 2-ethylhexanoic acid ($C_8$), nonanoic acid ($C_9$), decanoic acid ($C_{10}$), dodecanoic acid ($C_{12}$), tetradecanoic acid ($C_{14}$), hexadecanoic acid ($C_{16}$), heptadecanoic acid ($C_{17}$), octadecanoic acid ($C_{18}$), eicosanoic acid ($C_{20}$), docosanoic acid ($C_{22}$), tetracosanoic acid ($C_{24}$), hexacosanoic acid ($C_{26}$), octacosanoic acid ($C_{28}$), triacontanoic acid ($C_{30}$), as well as isomers thereof which are not described above.

The fatty acid may also be an unsaturated fatty acid. Examples of unsaturated fatty acids include $C_3$-$C_{20}$ unsaturated fatty acids, such as monounsaturated fatty acids including crotonic acid ($C_4$), myristoleic acid ($C_{14}$), palmitoleic acid ($C_{16}$), oleic acid ($C_{18}$), elaidic acid ($C_{18}$), vaccenic acid ($C_{18}$), gadoleic acid ($C_{20}$) and eicosenoic acid ($C_{20}$),

di-unsaturated fatty acids including linolic acid ($C_{18}$) and eicosadienoic acid ($C_{20}$), tri-unsaturated fatty acids including linolenic acids, such as α-linolenic acid ($C_{18}$) and γ-linolenic acid ($C_{18}$), pinolenic acid ($C_{18}$), eleostearic acids, such as α-eleostearic acid ($C_{18}$) and β-eleostearic acid ($C_{18}$), Mead acid ($C_{20}$), dihomo-γ-linolenic acid ($C_{20}$) and eicosatrienoic acid ($C_{20}$), tetra-unsaturated fatty acids including stearidonic acid ($C_{20}$), arachidonic acid ($C_{20}$) and eicosatetraenoic acid ($C_{20}$), penta-unsaturated fatty acids including bosseopentaenoic acid ($C_{18}$) and eicosapentaenoic acid ($C_{20}$), and partial hydrogen adducts thereof.

Considering the potential for degradation by oxidation and the like, the ester of pentaerythritol and a fatty acid is preferably an ester of pentaerythritol and a fatty acid, which is derived from a saturated fatty acid, i.e., an ester of pentaerythritol and a saturated fatty acid.

Also, from the viewpoint of lowering the water holding percentage, the ester of pentaerythritol and a fatty acid is preferably a diester, triester or tetraester, more preferably a triester or tetraester, and even more preferably a tetraester.

From the viewpoint of the IOB being from about 0.00 to about 0.60, in a tetraester of pentaerythritol and a fatty acid, the total number of carbons of the fatty acid composing the tetraester of the pentaerythritol and fatty acid, i.e. the total number of carbons of the $R^1C$, $R^2C$, $R^3C$ and $R^4C$ portions in formula (1), is preferably about 15 (the IOB is 0.60 when the total number of carbon atoms is 15).

Examples of tetraesters of pentaerythritol and fatty acids include tetraesters of pentaerythritol with hexanoic acid ($C_6$), heptanoic acid ($C_7$), octanoic acid ($C_8$), such as 2-ethylhexanoic acid ($C_8$), nonanoic acid ($C_9$), decanoic acid ($C_{10}$) and/or dodecanoic acid ($C_{12}$).

From the viewpoint of the IOB being from about 0.00 to about 0.60, in a triester of pentaerythritol and a fatty acid, the total number of carbons of the fatty acid composing the triester of the pentaerythritol and fatty acid, i.e. the total number of carbons of the $R^1C$, $R^2C$ and $R^3C$ portions in formula (2), is preferably about 19 or greater (the IOB is 0.58 when the number of carbon atoms is 19).

From the viewpoint of the IOB being from about 0.00 to about 0.60, in a diester of pentaerythritol and a fatty acid, the total number of carbons of the fatty acid composing the diester of the pentaerythritol and fatty acid, i.e. the total number of carbons of the $R^1C$ and $R^2C$ portion in formula (3), is preferably about 22 or greater (the IOB is 0.59 when the number of carbon atoms is 22).

From the viewpoint of the IOB being from about 0.00 to about 0.60, in a monoester of pentaerythritol and a fatty acid, the total number of carbons of the fatty acid composing the monoester of the pentaerythritol and fatty acid, i.e. the number of carbons of the $R^1C$ portion in formula (4), is preferably about 25 or greater (the IOB is 0.60 when the number of carbon atoms is 25).

The effects of double bonds, triple bonds, iso-branches and tert-branches are not considered in this calculation of the IOB (same hereunder).

Commercial products which are esters of pentaerythritol and fatty acids include UNISTAR H-408BRS and H-2408BRS-22 (mixed product) (both products of NOF Corp.).

[($a_2$) Ester of a chain hydrocarbon triol and at least one fatty acid]

Examples of esters of a chain hydrocarbon triol and at least one fatty acid include triesters of glycerin and fatty acids, represented by formula (5):

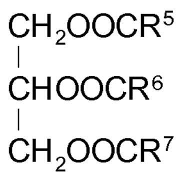

(5)

diesters of glycerin and fatty acids, represented by the following formula (6):

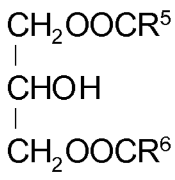

or

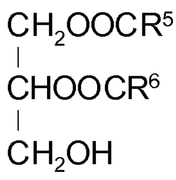

(6)

and monoesters of glycerin and fatty acids, represented by the following formula (7):

$$\begin{array}{l} CH_2OOCR^5 \\ | \\ CHOH \\ | \\ CH_2OH \end{array} \quad \text{or} \quad \begin{array}{l} CH_2OH \\ | \\ CHOOCR^5 \\ | \\ CH_2OH \end{array} \qquad (7)$$

wherein $R^5$-$R^7$ each represent a chain hydrocarbon.

The fatty acid consisting of the ester of glycerin and a fatty acid ($R^5COOH$, $R^6COOH$ and $R^7COOH$) is not particularly restricted so long as the ester of glycerin and a fatty acid satisfies the conditions for the kinematic viscosity, water holding percentage and weight-average molecular weight, and for example, there may be mentioned the fatty acids mentioned for the "($a_1$) Ester of a chain hydrocarbon tetraol and at least one fatty acid", namely saturated fatty acids and unsaturated fatty acids, and in consideration of the potential for degradation by oxidation and the like, the ester is preferably a glycerin and fatty acid ester, which is derived from a saturated fatty acid, i.e., an ester of glycerin and a saturated fatty acid.

Also, from the viewpoint of lowering the water holding percentage and result in greater hydrophobicity, the ester of glycerin and a fatty acid is preferably a diester or triester, and more preferably a triester.

A triester of glycerin and a fatty acid is also known as a triglyceride, and examples include triesters of glycerin and octanoic acid ($C_8$), triesters of glycerin and decanoic acid ($C_{10}$), triesters of glycerin and dodecanoic acid ($C_{12}$), triesters of glycerin and 2 or 3 different fatty acids, and mixtures threreof.

Examples of triesters of glycerin and 2 or more fatty acids include triesters of glycerin with octanoic acid ($C_8$) and decanoic acid ($C_{10}$), triesters of glycerin with octanoic acid ($C_8$), decanoic acid ($C_{10}$) and dodecanoic acid ($C_{12}$), and triesters of glycerin with octanoic acid ($C_8$), decanoic acid ($C_{10}$), dodecanoic acid ($C_{12}$), tetradecanoic acid ($C_{14}$), hexadecanoic acid ($C_{16}$) and octadecanoic acid ($C_{18}$).

In order to obtain a melting point of about 45°C or less, preferred triesters of glycerin and fatty acids are those with about 40 or less as the total number of carbons of the fatty acid consisting of the triester of glycerin and the fatty acid, i.e., the total number of carbons of the $R^5C$, $R^6C$ and $R^7C$ sections in formula (5).

From the viewpoint of the IOB being from about 0.00 to about 0.60, in a triester of glycerin and a fatty acid, the total number of carbons of the fatty acid composing the triester of the glycerin and fatty acid, i.e. the total number of carbons of the $R^5C$, $R^6C$ and $R^7C$ portions in formula (5), is preferably about 12 or greater (the IOB is 0.60 when the total number of carbon atoms is 12).

Triesters of glycerin and fatty acids, being aliphatic and therefore potential constituent components of the human body, are preferred from the viewpoint of safety.

Commercial products of triesters of glycerin and fatty acids include tri-coconut fatty acid glycerides, NA36, PANACET 800, PANACET 800B and PANACET 810S, and tri-C2L oil fatty acid glycerides and tri-CL oil fatty acid glycerides (all products of NOF Corp.).

A diester of glycerin and a fatty acid is also known as a diglyceride, and examples include diesters of glycerin and decanoic acid ($C_{10}$), diesters of glycerin and dodecanoic acid ($C_{12}$), diesters of glycerin and hexadecanoic acid ($C_{16}$), diesters of glycerin and 2 or more different fatty acids, and mixtures thereof.

From the viewpoint of the IOB being from about 0.00 to about 0.60, in a diester of glycerin and a fatty acid, the total number of carbons of the fatty acid composing the diester of the glycerin and fatty acid, i.e. the total number of carbons of the $R^5C$ and $R^6C$ portions in formula (6), is preferably about 16 or greater (the IOB is 0.58 when the total number of carbon atoms is 16).

Monoesters of glycerin and fatty acids are also known as monoglycerides, and examples include glycerin and octadecanoic acid ($C_{18}$) monoester, and glycerin and docosanoic acid ($C_{22}$) monoester.

From the viewpoint of the IOB being from about 0.00 to about 0.60, in a monoester of glycerin and a fatty acid, the total number of carbons of the fatty acid composing the monoester of the glycerin and fatty acid, i.e. the number of carbons of the $R^5C$ portion in formula (7), is preferably about 19 or greater (the IOB is 0.59 when the number of carbon atoms is 19).

[($a_3$) Ester of a chain hydrocarbon diol and at least one fatty acid]

Examples of an ester of a chain hydrocarbon diol and at least one fatty acid include monoesters and diesters of fatty acids with $C_2$-$C_6$ chain hydrocarbon diols, such as $C_2$-$C_6$ glycols, including ethylene glycol, propylene glycol, butylene glycol, pentylene glycol and hexylene glycol.

Specifically, examples of an ester of a chain hydrocarbon diol and at least one fatty acid include diesters of $C_2$-$C_6$ glycols and fatty acids, represented by the following formula (8):

$R^8COOC_kH_{2k}OCOR^9$ (8)

wherein k represents an integer of 2-6, and $R^8$ and $R^9$ each represent a chain hydrocarbon, and monoesters of $C_2$-$C_6$ glycols and fatty acids, represented by the following formula (9):

$R^8COOC_kH_{2k}OH$ (9)

wherein k represents an integer of 2-6, and $R^8$ is a chain hydrocarbon.

The fatty acid to be esterified in an ester of a $C_2$-$C_6$ glycol and a fatty acid (corresponding to $R^8COOH$ and $R^9COOH$ in formula (8) and formula (9)) is not particularly restricted so long as the ester of the $C_2$-$C_6$ glycol and fatty acid satisfies the conditions for the kinematic viscosity, water holding percentage and weight-average molecular weight, and for example, there may be mentioned the fatty acids mentioned above for the "($a_1$) Ester of a chain hydrocarbon tetraol and at least one fatty acid", namely saturated fatty acids and unsaturated fatty acids, and in consideration of the potential for degradation by oxidation and the like, it is preferably a saturated fatty acid.

From the viewpoint of the IOB being from about 0.00 to about 0.60, in a diester of butylene glycol represented by formula (8) (k = 4) and a fatty acid, the total number of carbons of the $R^8C$ and $R^9C$ portions is preferably about 6 or greater (the IOB is 0.60 when the total number of carbon atoms is 6).

From the viewpoint of the IOB being from about 0.00 to about 0.60, in a monoester of ethylene glycol represented by formula (9) (k = 2) and a fatty acid, the number of carbons of the $R^8C$ portion is preferably about 12 or greater (the IOB is 0.57 when the number of carbon atoms is 12).

Considering the potential for degradation by oxidation and the like, the ester of the $C_2$-$C_6$ glycol and fatty acid is preferably a $C_2$-$C_6$ glycol and fatty acid ester derived from a saturated fatty acid, or in other words, an ester of a $C_2$-$C_6$ glycol and a saturated fatty acid.

Also, from the viewpoint of lowering the water holding percentage, the ester of the $C_2$-$C_6$ glycol and fatty acid is preferably a glycol and fatty acid ester derived from a glycol with a greater number of carbons, such as an ester of a glycol and a fatty acid derived from butylene glycol, pentylene glycol or hexylene glycol.

Also, from the viewpoint of lowering the water holding percentage, the ester of a $C_2$-$C_6$ glycol and fatty acid is preferably a diester.

Examples of commercial products of esters of $C_2$-$C_6$ glycols and fatty acids include COMPOL BL and COMPOL BS (both products of NOF Corp.).

[(B) Ether of (B1) a compound having a chain hydrocarbon moiety and 2-4 hydroxyl groups substituting for hydrogens on the chain hydrocarbon moiety and (B2) a compound having a chain hydrocarbon moiety and 1 hydroxyl group substituting for a hydrogen on the chain hydrocarbon moiety]

In the (B) ether of (B1) a compound having a chain hydrocarbon moiety and 2-4 hydroxyl groups substituting for hydrogens on the chain hydrocarbon moiety and (B2) a compound having a chain hydrocarbon moiety and 1 hydroxyl group substituting for a hydrogen on the chain hydrocarbon moiety (hereunder also referred to as "compound (B)"), it is not necessary for all of the hydroxyl groups to be etherified so long as the kinematic viscosity, water holding percentage and weight-average molecular weight are within the aforementioned ranges.

Examples of (B1) a compound having a chain hydrocarbon moiety and 2-4 hydroxyl groups substituting for hydrogens on the chain hydrocarbon moiety (hereunder also referred to as "compound (B1)") include those mentioned for "compound (A)" as compound (A1), such as pentaerythritol, glycerin and glycol.

Examples of (B2) a compound having a chain hydrocarbon moiety and 1 hydroxyl group substituting for a hydrogen on the chain hydrocarbon moiety (hereunder also referred to as "compound (B2)") include compounds wherein 1 hydrogen on the hydrocarbon is substituted with 1 hydroxyl group (-OH), such as aliphatic monohydric alcohols, including saturated aliphatic monohydric alcohols and unsaturated aliphatic monohydric alcohols.

Examples of saturated aliphatic monohydric alcohols include $C_1$-$C_{20}$ saturated aliphatic monohydric alcohols, such as methyl alcohol ($C_1$) ($C_1$ representing the number of carbon atoms, same hereunder), ethyl alcohol ($C_2$), propyl alcohol ($C_3$) and isomers thereof, including isopropyl alcohol ($C_3$), butyl alcohol ($C_4$) and isomers thereof, including sec-butyl alcohol ($C_4$) and tert-butyl alcohol ($C_4$), pentyl alcohol ($C_5$), hexyl alcohol ($C_6$), heptyl alcohol ($C_7$), octyl alcohol ($C_8$) and isomers thereof, including 2-ethylhexyl alcohol ($C_8$), nonyl alcohol ($C_9$), decyl alcohol ($C_{10}$), dodecyl alcohol ($C_{12}$), tetradecyl alcohol ($C_{14}$), hexadecyl alcohol ($C_{16}$), heptadecyl alcohol ($C_{17}$), octadecyl alcohol ($C_{18}$) and eicosyl alcohol ($C_{20}$), as well as their isomers other than those mentioned.

Unsaturated aliphatic monohydric alcohols include those wherein 1 C-C single bond of a saturated aliphatic monohydric alcohol mentioned above is replaced with a C=C double bond, such as oleyl alcohol, and for example, such alcohols are commercially available by New Japan Chemical Co., Ltd. as the RIKACOL Series and UNJECOL Series.

Examples for compound (B) include ($b_1$) an ether of a chain hydrocarbon tetraol and at least one aliphatic

monohydric alcohol, such as monoethers, diethers, triethers and tetraethers, preferably diethers, triethers and tetraethers, more preferably triethers and tetraethers and even more preferably tetraethers, ($b_2$) an ether of a chain hydrocarbon triol and at least one aliphatic monohydric alcohol, such as monoethers, diethers and triethers, preferably diethers and triethers and more preferably triethers, and ($b_3$) an ether of a chain hydrocarbon diol and at least one aliphatic monohydric alcohol, such as monoethers and diethers, and preferably diethers.

Examples of an ether of a chain hydrocarbon tetraol and at least one aliphatic monohydric alcohol include tetraethers, triethers, diethers and monoethers of pentaerythritol and aliphatic monohydric alcohols, represented by the following formulas (10)-(13):

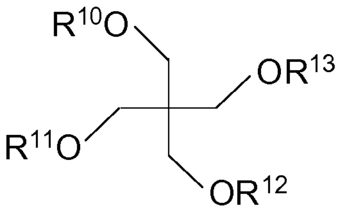 (10)

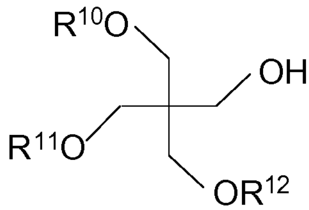 (11)

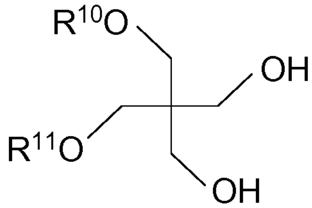 (12)

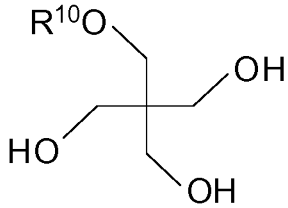 (13)

wherein $R^{10}$-$R^{13}$ each represent a chain hydrocarbon.

Examples of an ether of a chain hydrocarbon triol and at least one aliphatic monohydric alcohol include triethers, diethers and monoethers of glycerin and aliphatic monohydric alcohols, represented by the following formulas (14)-(16):

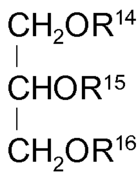 (14)

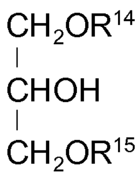 or 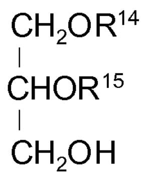 (15)

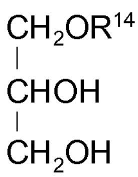 or 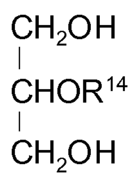 (16)

wherein $R^{14}$-$R^{16}$ each represent a chain hydrocarbon.

Examples of an ether of a chain hydrocarbon diol and at least one aliphatic monohydric alcohol include diethers of $C_2$-$C_6$ glycols and aliphatic monohydric alcohols, represented by the following formula (17):

$$R^{17}OC_nH_{2n}OR^{18} \qquad (17)$$

wherein n is an integer of 2-6, and $R^{17}$ and $R^{18}$ are each a chain hydrocarbon,
and monoethers of $C_2$-$C_6$ glycols and aliphatic monohydric alcohols, represented by the following formula (18) :

$R^{17}OC_nH_{2n}OH$ (18)

wherein n is an integer of 2-6, and $R^{17}$ is a chain hydrocarbon.

From the viewpoint of the IOB being between about 0.00 and about 0.60, in a tetraether of pentaerythritol and an aliphatic monohydric alcohol, the total number of carbon atoms of the aliphatic monohydric alcohol composing the tetraether of pentaerythritol and the aliphatic monohydric alcohol, i.e. the total number of carbon atoms of the $R^{10}$, $R^{11}$, $R^{12}$ and $R^{13}$ portions in formula (10), is preferably about 4 or greater (the IOB is 0.44 when the total number of carbon atoms is 4).

From the viewpoint of the IOB being between about 0.00 and about 0.60, in a triether of pentaerythritol and an aliphatic monohydric alcohol, the total number of carbon atoms of the aliphatic monohydric alcohol composing the triether of pentaerythritol and the aliphatic monohydric alcohol, i.e. the total number of carbon atoms of the $R^{10}$, $R^{11}$ and $R^{12}$ portions in formula (11), is preferably about 9 or greater (the IOB is 0.57 when the total number of carbon atoms is 9).

From the viewpoint of the IOB being between about 0.00 and about 0.60, in a diether of pentaerythritol and an aliphatic monohydric alcohol, the total number of carbon atoms of the aliphatic monohydric alcohol composing the diether of pentaerythritol and the aliphatic monohydric alcohol, i.e. the total number of carbon atoms of the $R^{10}$ and $R^{11}$ portions in formula (12), is preferably about 15 or greater (the IOB is 0.60 when the total number of carbon atoms is 15).

From the viewpoint of the IOB being between about 0.00 and about 0.60, in a monoether of pentaerythritol and an aliphatic monohydric alcohol, the number of carbon atoms of the aliphatic monohydric alcohol composing the monoether of pentaerythritol and the aliphatic monohydric alcohol, i.e. the number of carbon atoms of the $R^{10}$ portion in formula (13), is preferably about 22 or greater (the IOB is 0.59 when the number of carbon atoms is 22).

From the viewpoint of the IOB being between about 0.00 and about 0.60, in a triether of glycerin and an aliphatic monohydric alcohol, the total number of carbon atoms of the aliphatic monohydric alcohol composing the triether of glycerin and the aliphatic monohydric alcohol, i.e. the total number of carbon atoms of the $R^{14}$, $R^{15}$ and $R^{16}$ portions in formula (14), is preferably about 3 or greater (the IOB is 0.50 when the total number of carbon atoms is 3).

From the viewpoint of the IOB being between about 0.00 and about 0.60, in a diether of glycerin and an aliphatic monohydric alcohol, the total number of carbon atoms of the aliphatic monohydric alcohol composing the diether of glycerin and the aliphatic monohydric alcohol, i.e. the total number of carbon atoms of the $R^{14}$ and $R^{15}$ portions in formula (15), is preferably about 9 or greater (the IOB is 0.58 when the total number of carbon atoms is 9).

From the viewpoint of the IOB being between about 0.00 and about 0.60, in a monoether of glycerin and an aliphatic monohydric alcohol, the number of carbon atoms of the aliphatic monohydric alcohol composing the monoether of glycerin and the aliphatic monohydric alcohol, i.e. the number of carbon atoms of the $R^{14}$ portion in formula (16), is preferably 16 or greater (the IOB is 0.58 when the number of carbon atoms is 16).

From the viewpoint of the IOB being from about 0.00 to about 0.60, in a diether of butylene glycol represented by formula (17) (n = 4) and an aliphatic monohydric alcohol, the total number of carbon atoms of the $R^{17}$ and $R^{18}$ portions is preferably about 2 or greater (the IOB is 0.33 when the total number of carbon atoms is 2) .

From the viewpoint of the IOB being from about 0.00 to about 0.60, in a monoether of ethylene glycol represented by formula (18) (n = 2) and an aliphatic monohydric alcohol, the number of carbon atoms of the $R^{17}$ portion is preferably about 8 or greater (the IOB is 0.60 when the number of carbon atoms is 8).

Compound (B) may be produced by dehydrating condensation of compound (B1) and compound (B2) in the presence of an acid catalyst.

[(C) Ester of (C1) a carboxylic acid, hydroxy acid, alkoxy acid or oxoacid comprising a chain hydrocarbon moiety and 2-4 carboxyl groups substituting for hydrogens on the chain hydrocarbon moiety and (C2) a compound having a chain hydrocarbon moiety and 1 hydroxyl group substituting for a hydrogen on the chain hydrocarbon moiety]

In the (C) ester of (C1) a carboxylic acid, hydroxy acid, alkoxy acid or oxoacid comprising a chain hydrocarbon moiety and 2-4 carboxyl groups substituting for hydrogens on the chain hydrocarbon moiety and (C2) a compound having a chain hydrocarbon moiety and 1 hydroxyl group substituting for a hydrogen on the chain hydrocarbon moiety (hereunder also referred to as "compound (C)"), it is not necessary for all of the carboxyl groups to be esterified so long as the kinematic viscosity, water holding percentage and weight-average molecular weight are within the aforementioned ranges.

Examples of (C1) a carboxylic acid, hydroxy acid, alkoxy acid or oxoacid comprising a chain hydrocarbon moiety and 2-4 carboxyl groups substituting for hydrogens on the chain hydrocarbon moiety (hereunder also referred to as "compound (C1)") include chain hydrocarbon carboxylic acids with 2-4 carboxyl groups, such as chain hydrocarbon dicarboxylic acids including alkanedicarboxylic acids, such as ethanedioic acid, propanedioic acid, butanedioic acid, pentanedioic acid, hexanedioic acid, heptanedioic acid, octanedioic acid, nonanedioic acid and decanedioic acid, chain hydrocarbon tricarboxylic acids, including alkanetricarboxylic acids, such as propanetrioic acid, butanetrioic acid, pentanetrioic acid, hexanetrioic acid, heptanetrioic acid, octanetrioic acid, nonanetrioic acid and decanetrioic acid, and chain hydrocarbon tetracarboxylic acids, including alkanetetracarboxylic acids, such as butanetetraoic acid, pentanetetraoic acid, hexanetetraoic acid, heptanetetraoic acid, octanetetraoic acid, nonanetetraoic acid and decanetetraoic acid.

Compound (C1) includes chain hydrocarbon hydroxy acids with 2-4 carboxyl groups, such as malic acid, tartaric acid, citric acid and isocitric acid, chain hydrocarbon alkoxy acids with 2-4 carboxyl groups, such as O-acetylcitric acid, and chain hydrocarbon oxoacids with 2-4 carboxyl groups.

(C2) Compound having a chain hydrocarbon moiety and 1 hydroxyl group substituting for a hydrogen on the chain hydrocarbon moiety includes those mentioned for "compound (B)", such as aliphatic monohydric alcohols.

Compound (C) may be ($c_1$) an ester, for example a monoester, diester, triester or tetraester, preferably a diester, triester or tetraester, more preferably a triester or tetraester and even more preferably a tetraester, of a chain hydrocarbon tetracarboxylic acid, hydroxy acid, alkoxy acid or oxoacid with 4 carboxyl groups, and at least one aliphatic monohydric alcohol, ($c_2$) an ester, for example, a monoester, diester or triester, preferably a diester or triester and more preferably a triester, of a chain hydrocarbon tricarboxylic acid, hydroxy acid, alkoxy acid or oxoacid with 3 carboxyl groups, and at least one aliphatic monohydric alcohol, or ($c_3$) an ester, for example, a monoester or diester, and preferably a diester, of a chain hydrocarbon dicarboxylic acid, hydroxy acid, alkoxy acid or oxoacid with 2 carboxyl groups, and at least one aliphatic monohydric alcohol.

Examples for compound (C) include dioctyl adipate, and tributyl O-acetylcitrate, of which commercially available products exist.

[(D) Compound having a chain hydrocarbon moiety and one bond selected from the group consisting of an ether bond (-O-), carbonyl bond (-CO-), ester bond (-COO-) and carbonate bond (-OCOO-) inserted between a C-C single bond of the chain hydrocarbon moiety]

The (D) compound having a chain hydrocarbon moiety and one bond selected from the group consisting of an ether bond (-O-), carbonyl bond (-CO-), ester bond (-COO-) and carbonate bond (-OCOO-) inserted between a C-C single bond of the chain hydrocarbon moiety (hereunder also referred to as "compound (D)") may be ($d_1$) an ether of an aliphatic monohydric alcohol and an aliphatic monohydric alcohol, ($d_2$) a dialkyl ketone, ($d_3$) an ester of a fatty acid and an aliphatic monohydric alcohol, or ($d_4$) a dialkyl carbonate.

[($d_1$) Ether of an aliphatic monohydric alcohol and an aliphatic monohydric alcohol]

Ethers of an aliphatic monohydric alcohol and an aliphatic monohydric alcohol include compounds having the following formula (19):

$$R^{19}OR^{20} \qquad (19)$$

wherein $R^{19}$ and $R^{20}$ each represent a chain hydrocarbon.

The aliphatic monohydric alcohol consisting of the ether (corresponding to $R^{19}OH$ and $R^{20}OH$ in formula (19)) is not particularly restricted so long as the ether satisfies the conditions for the kinematic viscosity, water holding percentage and weight-average molecular weight, and for example, it may be one of the aliphatic monohydric alcohols mentioned for "compound (B)".

[($d_2$) Dialkyl ketone]

The dialkyl ketone may be a compound of the following formula (20):

$$R^{21}COR^{22} \qquad (20)$$

wherein $R^{21}$ and $R^{22}$ are each an alkyl group.

The dialkyl ketone may be a commercially available product, or it may be obtained by a known method, such as by oxidation of a secondary alcohol with chromic acid or the like.

[($d_3$) Ester of a fatty acid and an aliphatic monohydric alcohol]

Examples of esters of a fatty acid and an aliphatic monohydric alcohol include compounds having the following formula (21):

$R^{23}COOR^{24}$ (21)

wherein $R^{23}$ and $R^{24}$ each represent a chain hydrocarbon.

Examples of fatty acids consisting of these esters (corresponding to $R^{23}COOH$ in formula (21)) include the fatty acids mentioned for the "($a_1$) an ester of a chain hydrocarbon tetraol and at least one fatty acids", and specifically these include saturated fatty acids and unsaturated fatty acids, with saturated fatty acids being preferred in consideration of the potential for degradation by oxidation and the like. The aliphatic monohydric alcohol consisting of the ester (corresponding to $R^{24}OH$ in formula (21)) may be one of the aliphatic monohydric alcohols mentioned for "compound (B)".

Examples of esters of such fatty acids and aliphatic monohydric alcohols include esters of dodecanoic acid ($C_{12}$) and dodecyl alcohol ($C_{12}$) and esters of tetradecanoic acid ($C_{14}$) and dodecyl alcohol ($C_{12}$), and examples of commercial products of esters of such fatty acids and aliphatic monohydric alcohols include ELECTOL WE20 and ELECTOL WE40 (both products of NOF Corp.).

[($d_4$) Dialkyl carbonate]

The dialkyl carbonate may be a compound of the following formula (22):

$R^{25}OC(=O)OR^{26}$ (22)

wherein $R^{25}$ and $R^{26}$ are each an alkyl group.

The dialkyl carbonate may be a commercially available product, or it may be synthesized by reaction between phosgene and an alcohol, reaction between formic chloride and an alcohol or alcoholate, or reaction between silver carbonate and an alkyl iodide.

From the viewpoint of the water holding percentage and vapor pressure, the weight-average molecular weight is preferably about 100 or greater and more preferably about 200 or greater, for ($d_1$) an ether of an aliphatic monohydric alcohol and an aliphatic monohydric alcohol, ($d_2$) a dialkyl ketone, ($d_3$) an ester of a fatty acid and an aliphatic monohydric alcohol, and ($d_4$) a dialkyl carbonate.

If the total number of carbon atoms is about 8 in a ($d_2$) dialkyl ketone, the melting point will be approximately -50°C and the vapor pressure will be about 230 Pa at 20°C, in the case of 5-nonanone, for example.

[(E) Polyoxy $C_3$-$C_6$ alkylene glycol, or alkyl ester or alkyl ether thereof]

The (E) polyoxy $C_3$-$C_6$ alkylene glycol, or alkyl ester or alkyl ether thereof (hereunder also referred to as "compound (E)") may be ($e_1$) a polyoxy $C_3$-$C_6$ alkylene glycol, ($e_2$) an ester of a polyoxy $C_3$-$C_6$ alkylene glycol and at least one fatty acid, or ($e_3$) an ether of a polyoxy $C_3$-$C_6$ alkylene glycol and at least one aliphatic monohydric alcohol. These will now be explained.

[($e_1$) Polyoxy $C_3$-$C_6$ alkylene glycol]

Polyoxy $C_3$-$C_6$ alkylene glycols refer to i) homopolymers having one unit selected from the group consisting of oxy $C_3$-$C_6$ alkylene units, such as oxypropylene unit, oxybutylene unit, oxypentylene unit and oxyhexylene unit and having hydroxyl groups at both ends, ii) block copolymers having 2 or more units selected from oxy $C_3$-$C_6$ alkylene units described above and oxyhexylene unit and having hydroxyl groups at both ends, or iii) random copolymers having 2 or more units selected from oxy $C_3$-$C_6$ alkylene units described above and having hydroxyl groups at both ends.

The polyoxy $C_3$-$C_6$ alkylene glycol can be represented by the following formula (23):

$HO-(C_mH_{2m}O)_n-H$ (23)

wherein m represents an integer of 3-6.

The present inventors have found that with polypropylene glycol (corresponding to a homopolymer of formula (23) where m = 3), the condition for the water holding percentage is not satisfied when the weight-average molecular weight is less than about 1,000. Therefore, polypropylene glycol homopolymer is not included in the scope of the blood slipping agent described above, and propylene glycol should be included in the ($e_1$) polyoxy $C_3$-$C_6$ alkylene glycol only as a copolymer or random polymer with another glycol.

Incidentally, investigation by the present inventors suggests that with polyethylene glycol (corresponding to a homopolymer of formula (23) where m = 2), the condition for the kinematic viscosity and water holding percentage cannot be satisfied when the weight-average molecular weight is less than about 1,000.

From the viewpoint of the IOB being about 0.00 to about 0.60, when formula (23) is polybutylene glycol (a homopolymer where m = 4), for example, preferably n ≥ about 7 (when n = 7, the IOB is 0.57).

Examples of commercial products of poly $C_3$-$C_6$ alkylene glycols include UNIOL™ PB-500 and PB-700 (all products of NOF Corp.).

[($e_2$) Ester of a polyoxy $C_3$-$C_6$ alkylene glycol and at least one fatty acid]

Examples of an ester of a polyoxy $C_3$-$C_6$ alkylene glycol and at least one fatty acids include the polyoxy $C_3$-$C_6$ alkylene glycols mentioned for "($e_1$) Polyoxy $C_3$-$C_6$ alkylene glycol" in which one or both OH ends have been esterified with fatty acids, i.e. monoesters and diesters.

Examples of fatty acids to be esterified in the ester of a polyoxy $C_3$-$C_6$ alkylene glycol and at least one fatty acid include the fatty acids mentioned for the "($a_1$) Ester of a chain hydrocarbon tetraol and at least one fatty acid", and specifically these include saturated fatty acids and unsaturated fatty acids, with saturated fatty acids being preferred in consideration of the potential for degradation by oxidation and the like.

[($e_3$) Ether of a polyoxy $C_3$-$C_6$ alkylene glycol and at least one aliphatic monohydric alcohol]

Examples of an ether of a polyoxy $C_3$-$C_6$ alkylene glycols and at least one aliphatic monohydric alcohol include the polyoxy $C_3$-$C_6$ alkylene glycols mentioned for "($e_1$) Polyoxy $C_3$-$C_6$ alkylene glycol" wherein one or both OH ends have been etherified by an aliphatic monohydric alcohol, i.e. monoethers and diethers.

In an ether of a polyoxy $C_3$-$C_6$ alkylene glycol and at least one aliphatic monohydric alcohol, the aliphatic monohydric alcohol to be etherified may be an aliphatic monohydric alcohol among those mentioned for "compound (B) ".

[(F) Chain hydrocarbon]

Examples of chain hydrocarbons include ($f_1$) chain alkanes, such as straight-chain alkanes and branched chain alkanes. Straight-chain alkanes with melting points of about 45°C or less have up to about 22 carbon atoms, and at a vapor pressure of 1 atmosphere and about 0.01 Pa or less at 25°C, the number of carbon atoms is 13 or greater. Branched chain alkanes tend to have lower melting points than chain alkanes, given the same number of carbon atoms. Branched chain alkanes may therefore include those with 22 and more carbon atoms, even with melting points of below about 45°C.

Examples of commercially available hydrocarbon products include PARLEAM 6 (NOF Corp.).

In a nonwoven fabric according to one embodiment of this disclosure, the second region, third region and fourth region respectively have a blood slipping agent-containing second region, a blood slipping agent-containing third region and a blood slipping agent-containing fourth region, that contain a blood slipping agent.

In a nonwoven fabric according to another embodiment of this disclosure, the second region, third region and/or fourth region have a blood slipping agent-containing second region, blood slipping agent-containing third region and/or blood slipping agent-containing fourth region, that consist entirely of a blood slipping agent.

In a nonwoven fabric according to yet another embodiment of this disclosure, the second region, third region and/or fourth region have a blood slipping agent-containing second region, blood slipping agent-containing third region and/or blood slipping agent-containing fourth region comprising a blood slipping agent-containing composition that contains the blood slipping agent and at least one other component.

Such a blood slipping agent-containing composition will now be described.

[blood slipping agent-containing composition]

The blood slipping agent-containing composition contains a blood slipping agent and at least one other component. The other component is not particularly restricted so long as it does not inhibit the effect of the present disclosure, and it may be any one commonly employed in absorbent articles of the art, and especially top sheets.

Examples for the other component(s) include silicone oils, silicones, silicone-based resins and the like.

Examples for the other component(s) also include antioxidants, such as BHT (2,6-di-t-butyl-p-cresol), BHA (butylated hydroxyanisole) and propyl gallate.

Further examples for the other component(s) include vitamins, such as natural vitamins and synthetic vitamins. Examples of vitamins include water-soluble vitamins, such as group B vitamins, including vitamin $B_1$, vitamin $B_2$, vitamin $B_3$, vitamin $B_5$, vitamin $B_6$, vitamin $B_7$, vitamin $B_9$ and vitamin $B_{12}$, and vitamin C.

Other examples of vitamins include fat-soluble vitamins, such as group A vitamins, group D vitamins, group E vitamins and group K vitamins.

The derivatives of these vitamins are also included.

Examples for the other component(s) include amino acids, such as alanine, arginine, lysine, histidine, proline and hydroxyproline, and peptides.

Other examples for the other component(s) include zeolite, such as natural zeolite, examples of which include analcite, chabazite, heulandite, natrolite, stilbite and thomosonite, and synthetic zeolite.

Still other examples for the other component(s) include cholesterol, hyaluronic acid, lecithin and ceramide.

Yet other examples for the other component(s) include drugs, such as skin astringents, anti-pimple medications, anti-wrinkle agents, anti-cellulite agents, skin whiteners, antimicrobial agents and antifungal agents.

Examples of skin astringents include zinc oxide, aluminum sulfate, tannic acid and the like, and oil-soluble skin astringents, such as fat-soluble polyphenols. Fat-soluble polyphenols include natural fat-soluble polyphenols, such as barley extract, otogiriso extract, white deadnettle extract, chamomilla extract, burdock extract, salvia extract, linden extract, common lime extract, white birch extract, common horsetail extract, sage extract, salvia extract, walnut (*J. regia* L. var. *orientalis*) extract, hibiscus extract, loquat leaf extract, Miquel's linden extract, hop extract, common horse-chestnut extract and coix seed extract.

Examples of anti-pimple medications include salicylic acid, benzoyl peroxide, resorcinol, sulfur, erythromycin and zinc.

Examples of anti-wrinkle agents include lactic acid, salicylic acid, salicylic acid derivatives, glycolic acid, phytic acid, lipoic acid and lysophosphatidic acid.

Examples of anti-cellulite agents include xanthine compounds, such as aminophylline, caffeine, theophylline and theobromine.

Examples of skin whiteners include niacinamide, kojic acid, arbutin, glucosamine and its derivatives, phytosterol derivatives, and ascorbic acid and its derivatives, as well as mulberry extract and placenta extract.

Examples for the other component(s) also include anti-inflammatory components, pH regulators, antimicrobial agents, humectants, aromatics, pigments, dyes, pigments and plant extracts. Examples of anti-inflammatory components include naturally-derived anti-inflammatory drugs, such as peony, golden grass, otogiriso, chamomile, licorice, peach leaf, Japanese mugwort and perilla extract, and synthetic anti-inflammatory drugs, such as allantoin and dipotassium glycyrrhizinate.

Examples of pH regulators include those that keep the skin weakly acidic, such as malic acid, succinic acid, citric acid, tartaric acid and lactic acid.

Titanium oxide is an example of a pigment.

The blood slipping agent-containing composition contains the blood slipping agent and the one or more other components at preferably about 50 to about 99 mass% and about 1 to about 50 mass%, respectively, more preferably about 60 to about 99 mass% and about 1 to about 40 mass%, respectively, even more preferably about 70 to about 99 mass% and about 1 to about 30 mass%, respectively, yet more preferably about 80 to about 99 mass% and about 1 to about 20 mass%, respectively, even yet more preferably about 90 to 99 mass% and about 1 to about 10 mass%, respectively, and even yet more preferably about 95 to 99 mass% and about 1 to about 5 mass%, respectively. These ranges are from the viewpoint of the effect of the present disclosure.

The blood slipping agent-containing composition preferably contains a surfactant in not greater than the amount from hydrophilicizing treatment of the top sheet or second sheet. More specifically, the blood slipping agent-containing composition contains a surfactant in a basis weight range of preferably about 0.0 to about 1.0 g/m$^2$, more preferably about 0.0 to about 0.8 g/m$^2$, even more preferably about 0.1 to about 0.5 g/m$^2$, and yet more preferably about 0.1 to about 0.3 g/m$^2$.

This is because when the amount of surfactant is increased, menstrual blood will tend to be retained in the top sheet. The surfactant, incidentally, has no water holding percentage. This is because there is no layer of the substance to be measured, due to admixture with water.

The blood slipping agent-containing composition contains water in a basis weight range of preferably about 0.0 to about 1.0 g/m$^2$, more preferably about 0.0 to about 0.8 g/m$^2$, even more preferably about 0.1 to about 0.5 g/m$^2$, and yet more preferably about 0.1 to about 0.3 g/m$^2$.

Since water lowers the absorption performance of the absorbent article, the amount is preferably low.

Similar to the blood slipping agent, the blood slipping agent-containing composition, as a composition, has at 40°C, a kinematic viscosity of preferably about 0 to about 80 mm$^2$/s, more preferably a kinematic viscosity of about 1 to about 70 mm$^2$/s, even more preferably a kinematic viscosity of about 3 to about 60 mm$^2$/s, yet more preferably a kinematic viscosity of about 5 to about 50 mm$^2$/s, and even yet more preferably a kinematic viscosity of about 7 to about 45 mm$^2$/s.

If the kinematic viscosity of the blood slipping agent-containing composition exceeds 80 mm$^2$/s, the viscosity will increase, and the blood slipping agent composition may not slide down into the interior of the absorbent article as easily with menstrual blood that has reached the skin contact surface of the top sheet.

When the blood slipping agent-containing composition contains a component that is miscible with the blood slipping agent, as at least one other component, the other component preferably has a weight-average molecular weight of less than about 1000, and more preferably a weight-average molecular weight of less than about 900. This is because,

if the weight-average molecular weight is about 1000 or higher, tack may result in the blood slipping agent-containing composition itself, tending to create a feeling of unpleasantness for the wearer. If the weight-average molecular weight increases, the viscosity of the blood slipping agent-containing composition will tend to increase, and it will therefore be difficult to lower the viscosity of the blood slipping agent composition by heating to a viscosity suitable for coating, and as a result, the blood slipping agent may need to be diluted with a solvent.

The blood slipping agent-containing composition, as a composition, has a water holding percentage of about 0.01 to about 4.0 mass%, preferably it has a water holding percentage of about 0.02 to about 3.5 mass%, more preferably it has a water holding percentage of about 0.03 to about 3.0 mass%, even more preferably it has a water holding percentage of about 0.04 to about 2.5 mass%, and yet more preferably it has a water holding percentage of about 0.05 to about 2.0 mass%.

A low water holding percentage value will tend to lower the affinity between the blood slipping agent composition and menstrual blood, thus inhibiting it from sliding down into the interior of the absorbent article with menstrual blood that has reached the skin contact surface of the top sheet.

When the blood slipping agent-containing composition contains solid matter, it is preferably removed by filtration for measurement of the kinematic viscosity and water holding percentage.

The blood slipping agent or blood slipping agent-containing composition may, if desired, be applied as a coating solution containing a volatile solvent, such as an alcohol-based solvent, ester-based solvent or aromatic solvent. If the coating solution includes a volatile solvent, the viscosity of the coating solution containing the blood slipping agent or blood slipping agent-containing composition will be lowered, thereby allowing the application steps to be simplified, facilitating application and making heating during application unnecessary.

[Nonwoven fabric and method for producing absorbent article]

The nonwoven fabric of this disclosure is produced by forming a nonwoven fabric that is to be coated with a blood slipping agent, and then coating a blood slipping agent onto the formed nonwoven fabric, according to the method described in Japanese Unexamined Patent Publication No. 2008-25078, for example. For example, the nonwoven fabric 5 shown in Fig. 1 is produced according to the "first example" of Japanese Unexamined Patent Publication No. 2008-25078, and the nonwoven fabric 5 shown in Fig. 3 is produced according to the "second example" of the same.

There are no particular restrictions on the method of applying the blood slipping agent or blood slipping agent-containing composition, or the coating solution containing it, and if necessary the blood slipping agent or blood slipping agent-containing composition or the coating solution containing it may be heated, and a coating applicator, for example a non-contact coater, such as a spiral coater, curtain coater, spray coater or dip coater, or a contact coater, may be used for application of the blood slipping agent or blood slipping agent-containing composition or the coating solution containing it. The coating applicator is preferably a non-contact coater, from the viewpoint of uniformly dispersing the droplet or particulate modifying agent throughout, and from the viewpoint of not causing damage in the material.

The blood slipping agent or blood slipping agent-containing composition, or the coating solution containing it, may be coated directly, if it is a liquid at room temperature, or it may be heated to lower the viscosity, and when it is a solid at room temperature, it may be heated to liquefaction and coated from a control seam HMA (Hot Melt Adhesive) gun. By increasing the air pressure of the control seam HMA gun, it is possible to apply the blood slipping agent or blood slipping agent-containing composition as fine particulates.

The coating amount of the blood slipping agent or blood slipping agent-containing composition may be adjusted, for example, by increasing or reducing the amount of application from the control seam HMA gun.

The basis weights of the blood slipping agent in the second region, third region and fourth region can be modified in the following manner, for example.

(1) When the nonwoven fabric of this disclosure is to be produced according to Japanese Unexamined Patent Publication No. 2008-25078, the blood slipping agent and other components are blasted onto a fiber web through a blowing nozzle, together with hot air.
The blood slipping agent and other components blown from the blowing nozzle are repelled after striking the first region, and adhere onto the second region, third region and fourth region. As a result of the blasting, the basis weight of the blood slipping agent in the second region and fourth region, which are relatively closer to the blowing nozzle, is greater than the basis weight of the blood slipping agent in the fourth region which is relatively farther from the blowing nozzle.

(2) When the nonwoven fabric has a plurality of ridges (second regions, third regions and fourth regions) and a plurality of furrows (first regions), the blood slipping agent and other components are coated along the furrows (first regions) centering on the sides of the ridges (second regions and fourth regions), from a control seam HMA gun, for example.

(3) When the nonwoven fabric has a plurality of ridges and a plurality of furrows, masking tape is attached to the

center sections of the ridges (the third regions) and the blood slipping agent and other components are evenly coated over the entire nonwoven fabric, after which the masking tape is released.

An absorbent article including the nonwoven fabric of this disclosure can be produced by a method known in the technical field. For example, it can be produced by layering a liquid-permeable back sheet, an absorbent body and the aforementioned nonwoven fabric with an adhesive between them, and then cutting it into an absorbent article shape.

The fibers composing such a nonwoven fabric may be natural fibers or chemical fibers, with examples of natural fibers including cellulose, such as ground pulp and cotton, and examples of chemical fibers including regenerated cellulose, such as rayon and fibril rayon, semi-synthetic cellulose, such as acetate and triacetate, thermoplastic hydrophobic chemical fibers, and hydrophilicized thermoplastic hydrophobic chemical fibers.

Examples of thermoplastic hydrophobic chemical fibers include polyethylene (PE), polypropylene (PP) and polyethylene terephthalate (PET) monofilaments, and fibers composed of PE and PP graft polymers.

Liquid-impermeable back sheets include films comprising PE and PP, air-permeable resin films, air-permeable resin films bonded to spunbond or spunlace nonwoven fabrics, and multilayer nonwoven fabrics, such as SMS. In consideration of flexibility of the absorbent article, a low-density polyethylene (LDPE) film with a basis weight of about 15-30 g/m$^2$, for example, is preferred.

An absorbent article according to one embodiment of this disclosure includes a second sheet between the liquid-permeable top sheet and the absorbent body. The second sheet may be any of the same examples as for the liquid-permeable top sheet.

The first example of the absorbent body is one having an absorbent core covered with a core wrap.

Examples of components for the absorbent core include hydrophilic fibers, including cellulose, such as ground pulp or cotton, regenerated cellulose, such as rayon or fibril rayon, semi-synthetic cellulose, such as acetate or triacetate, particulate polymers, filamentous polymers, thermoplastic hydrophobic chemical fibers, and hydrophilicized thermoplastic hydrophobic chemical fibers, as well as combinations thereof. The component of the absorbent core may also be a super absorbent polymer, such as granules of a sodium acrylate copolymer or the like.

The core wrap is not particularly restricted so long as it is a substance that is liquid-permeable and with a barrier property that does not allow permeation of the polymer absorber, and it may be a woven fabric or nonwoven fabric, for example. The woven fabric or nonwoven fabric may be made of a natural fiber, chemical fiber, tissue, or the like.

A second example of the absorbent body is one formed from an absorbing sheet or polymer sheet, with a thickness of preferably about 0.3-5.0 mm. The absorbing sheet or polymer sheet may usually be used without any particular restrictions so long as it is one that can be used in an absorbent article, such as a sanitary napkin.

The side sheet may be any of the same examples as for the liquid-permeable top sheet.

The flap can be formed from a side sheet and a liquid-impermeable back sheet, and optionally it may have a reinforcing sheet, such as paper, between them.

The blood slipping agent or blood slipping agent-containing composition preferably does not occlude the voids between the fibers of the nonwoven fabric, and the blood slipping agent or blood slipping agent-containing composition may, for example, adhere as droplets or particulates on the surfaces of the fibers of the nonwoven fabric, or it may cover the surfaces of the fibers.

Furthermore, in order for the blood slipping agent or blood slipping agent-containing composition to slip down together with the absorbed menstrual blood, it preferably has a large surface area, and a blood slipping agent or blood slipping agent-containing composition present as droplets or particulates preferably has a small droplet/particle diameter.

An absorbent article according to another embodiment of this disclosure has a second sheet containing a blood slipping agent. An absorbent article according to yet another embodiment of this disclosure has an absorbent body containing a blood slipping agent.

A nonwoven fabric coated with a blood slipping agent or blood slipping agent-containing composition is preferably subjected to hydrophilicizing treatment. The hydrophilicizing treatment may involve coating the surfaces of the fibers of the nonwoven fabric with a hydrophilic agent, or mixing a hydrophilic agent with the synthetic resin used as the starting material for the nonwoven fabric.

This is because if the nonwoven fabric before coating of the blood slipping agent or blood slipping agent-containing composition is hydrophilic, there will be lipophilic regions due to the blood slipping agent and hydrophilic regions due to the hydrophilic agent, that are sparsely dispersed on the top sheet, and this will allow the blood slipping agent or blood slipping agent-containing composition to exhibit slipping performance and will also facilitate rapid migration of menstrual blood into the absorbent body.

The blood slipping agent or blood slipping agent-containing composition also has an effect as a lubricant. Thus the blood slipping agent or blood slipping agent-containing composition reduces friction between the fibers of the nonwoven fabric, and improves the flexibility of the nonwoven fabric as a whole.

An absorbent article according to a preferred embodiment of this disclosure may be one that is intended for absorption of blood, such as a sanitary napkin or panty liner.

The nonwoven fabric of this disclosure, and the absorbent article comprising the nonwoven fabric, differ from known absorbent articles containing skin care compositions, lotion compositions and the like, in that they do not need components, such as emollients or immobilizing agents, and therefore the nonwoven fabric according to one embodiment of this disclosure, and an absorbent article comprising the nonwoven fabric, do not contain an emollient and/or immobilizing agent.

Examples

The present disclosure will now be explained in fuller detail by examples, with the understanding that it is not meant to be limited to the examples.

[Example 1]

[Evaluation of rewetting rate and absorbent body migration rate]

A commercially available sanitary napkin having the shape shown in Fig. 5 (without three-dimensional gathers and not coated with a blood slipping agent) was prepared. The sanitary napkin was formed from a top sheet, formed of a hydrophilic agent-treated air-through nonwoven fabric (composite fiber composed of polyester and polyethylene terephthalate, basis weight: 35 g/m$^2$), a second sheet, formed of an air-through nonwoven fabric (composite fiber composed of polyester and polyethylene terephthalate, basis weight: 30 g/m$^2$), an absorbent body comprising pulp (basis weight: 150 to 450 g/m$^2$, increased at the center section), an acrylic super-absorbent polymer (basis weight: 15 g/m$^2$) and tissue as a core wrap, a water-repellent agent-treated side sheet, and a back sheet composed of a polyethylene film.

The blood slipping agents used for testing are listed below.

[($a_1$) Ester of chain hydrocarbon tetraol and at least one fatty acid]

• UNISTAR H-408BRS, product of NOF Corp.

Pentaerythritol tetra(2-ethylhexanoate), weight-average molecular weight: approximately 640

• UNISTAR H-2408BRS-22, product of NOF Corp.

Mixture of pentaerythritol tetra(2-ethylhexanoate) and neopentylglycol di(2-ethylhexanoate) (58:42 as weight ratio), weight-average molecular weight: approximately 520

[($a_2$) Ester of chain hydrocarbon triol and at least one fatty acid]

• Cetiol SB45DEO, Cognis Japan

Glycerin and fatty acid triester, with oleic acid or stearylic acid as the fatty acid.

• SOY42, product of NOF Corp.

Glycerin and fatty acid triester with $C_{14}$ fatty acid:$C_{16}$ fatty acid:$C_{18}$ fatty acid:$C_{20}$ fatty acid (including both saturated fatty acids and unsaturated fatty acids) at a mass ratio of about 0.2:11:88:0.8, weight-average molecular weight: 880

• Tri-C2L oil fatty acid glyceride, product of NOF Corp.

Glycerin and fatty acid triester with $C_8$ fatty acid:$C_{10}$ fatty acid:$C_{12}$ fatty acid at a weight ratio of about 37:7:56, weight-average molecular weight: approximately 570

• Tri-CL oil fatty acid glyceride, product of NOF Corp.

Glycerin and fatty acid triester with $C_8$ fatty acid:$C_{12}$ fatty acid at a weight ratio of about 44:56, weight-average molecular weight: approximately 570

• PANACET 810s, product of NOF Corp.

Glycerin and fatty acid triester with $C_8$ fatty acid:$C_{10}$ fatty acid at a mass ratio of about 85:15, weight-average molecular weight: approximately 480

• PANACET 800, product of NOF Corp.

Glycerin and fatty acid triester with octanoic acid ($C_8$) as the entire fatty acid portion, weight-average molecular weight: approximately 470

• PANACET 800B, product of NOF Corp.

Glycerin and fatty acid triester with 2-ethylhexanoic acid ($C_8$) as the entire fatty acid portion, weight-average molecular weight: approximately 470

• NA36, product of NOF Corp.

Glycerin and fatty acid triester with $C_{16}$ fatty acid:$C_{18}$ fatty acid:$C_{20}$ fatty acid (including both saturated fatty acids and unsaturated fatty acids) at a mass ratio of about 5:92:3, weight-average molecular weight: approximately 880

• Tri-coconut fatty acid glyceride, product of NOF Corp.

Glycerin and fatty acid triester with $C_8$ fatty acid:$C_{10}$ fatty acid:$C_{12}$ fatty acid:$C_{14}$ fatty acid:$C_{16}$ fatty acid (including both saturated fatty acids and unsaturated fatty acids) at a mass ratio of about 4:8:60:25:3, weight-average molecular weight: 670

• Caprylic acid diglyceride, product of NOF Corp.

Glycerin and fatty acid diester with octanoic acid as the fatty acid, weight-average molecular weight: approximately 340

[($a_3$) Ester of a chain hydrocarbon diol and at least one fatty acid]

• UNISTAR H-208BRS, product of NOF Corp.

Neopentyl glycol di(2-ethylhexanoate), weight-average molecular weight: approximately 360

• COMPOL BL, product of NOF Corp.

Dodecanoic acid ($C_{12}$) monoester of butylene glycol, weight-average molecular weight: approximately 270

• COMPOL BS, product of NOF Corp.

Octadecanoic acid ($C_{18}$) monoester of butylene glycol, weight-average molecular weight: approximately 350

[($c_2$) Ester of a chain hydrocarbon tricarboxylic acid, hydroxy acid, alkoxy acid or oxoacid with 3 carboxyl groups, and at least one aliphatic monohydric alcohol]

• Tributyl O-acetylcitrate, product of Tokyo Kasei Kogyo Co., Ltd.

Weight-average molecular weight: approximately 400

• Tributyl citrate, product of Tokyo Kasei Kogyo Co., Ltd.

Weight-average molecular weight: approximately 360

[($c_3$) Ester of a chain hydrocarbon dicarboxylic acid, hydroxy acid, alkoxy acid or oxoacid with 2 carboxyl groups, and at least one aliphatic monohydric alcohol]

• Dioctyl adipate, product of Wako Pure Chemical Industries, Ltd.

Weight-average molecular weight: approximately 380

[($d_3$) Ester of a fatty acid and an aliphatic monohydric alcohol]

• ELECTOL WE20, product of NOF Corp.

Ester of dodecanoic acid ($C_{12}$) and dodecyl alcohol ($C_{12}$), weight-average molecular weight: approximately 360

• ELECTOL WE40, product of NOF Corp.

Ester of tetradecanoic acid ($C_{14}$) and dodecyl alcohol ($C_{12}$), weight-average molecular weight: approximately 390

[($e_1$) Polyoxy $C_3$-$C_6$ alkylene glycol]

• UNIOL PB500, product of NOF Corp.

Polybutylene glycol, weight-average molecular weight: approximately 500

• UNIOL PB700, product of NOF Corp.

Polyoxybutylene polyoxypropylene glycol, weight-average molecular weight: approximately 700

[($f_1$) Chain alkane]

• PARLEAM 6, product of NOF Corp.

Branched chain hydrocarbon, produced by copolymerization of liquid isoparaffin, isobutene and n-butene followed by hydrogen addition, polymerization degree: approximately 5-10, weight-average molecular weight: approximately 330

[Other materials]

• NA50, product of NOF Corp.

Glycerin and fatty acid triester obtained by addition of hydrogen to NA36 for reduced proportion of double bonds from unsaturated fatty acid starting material, weight-average molecular weight: approximately 880

• (Caprylic acid/capric acid) monoglyceride, product of NOF Corp.

Glycerin and fatty acid monoester, with octanoic acid ($C_8$) and decanoic acid ($C_{10}$) at a mass ratio of about 85:15, weight-average molecular weight: approximately 220

• Monomuls 90-L2 lauric acid monoglyceride, product of Cognis Japan

• Isopropyl citrate, product of Tokyo Kasei Kogyo Co., Ltd.

Weight-average molecular weight: approximately 230

• Diisostearyl malate

Weight-average molecular weight: approximately 640

• UNIOL PB1000R, product of NOF Corp.

Polybutylene glycol, weight-average molecular weight: approximately 1,000

• UNIOL D-250, product of NOF Corp.

Polypropylene glycol, weight-average molecular weight: approximately 250

• UNIOL D-400, product of NOF Corp.

Polypropylene glycol, weight-average molecular weight: approximately 400

• UNIOL D-700, product of NOF Corp.

Polypropylene glycol, weight-average molecular weight: approximately 700

• UNIOL D-1000, product of NOF Corp.

Polypropylene glycol, weight-average molecular weight: approximately 1,000

• UNIOL D-1200, product of NOF Corp.

Polypropylene glycol, weight-average molecular weight: approximately 1,160

• UNIOL D-2000, product of NOF Corp.

Polypropylene glycol, weight-average molecular weight: approximately 2,030

• UNIOL D-3000, product of NOF Corp.

Polypropylene glycol, weight-average molecular weight: approximately 3,000

• UNIOL D-4000, product of NOF Corp.

Polypropylene glycol, weight-average molecular weight: approximately 4,000

•PEG1500, product of NOF Corp.

Polyethylene glycol, weight-average molecular weight: approximately 1,500-1,600

• WILBRITE cp9, product of NOF Corp.

Polybutylene glycol compound with OH groups at both ends esterified by hexadecanoic acid ($C_{16}$), weight-average molecular weight: approximately 1,150

• UNILUBE MS-70K, product of NOF Corp.

Stearyl ether of polypropylene glycol, approximately 15 repeating units, weight-average molecular weight: approximately 1,140

• NONION S-6, product of NOF Corp.

Polyoxyethylene monostearate, approximately 7 repeating units, weight-average molecular weight: approximately 880

• UNILUBE 5TP-300KB

Polyoxyethylene polyoxypropylene pentaerythritol ether, produced by addition of 5 mol of ethylene oxide and

65 mol of propylene oxide to 1 mol of pentaerythritol, weight-average molecular weight: 4,130

• WILBRITE s753, product of NOF Corp.

Polyoxyethylene polyoxypropylene polyoxybutylene glycerin, weight-average molecular weight: approximately 960

• UNIOL TG-330, product of NOF Corp.

Glyceryl ether of polypropylene glycol, approximately 6 repeating units, weight-average molecular weight: approximately 330

• UNIOL TG-1000, product of NOF Corp.

Glyceryl ether of polypropylene glycol, approximately 16 repeating units, weight-average molecular weight: approximately 1,000

• UNIOL TG-3000, product of NOF Corp.

Glyceryl ether of polypropylene glycol, approximately 16 repeating units, weight-average molecular weight: approximately 3,000

• UNIOL TG-4000, product of NOF Corp.

Glyceryl ether of polypropylene glycol, approximately 16 repeating units, weight-average molecular weight: approximately 4,000

• UNILUBE DGP-700, product of NOF Corp.

Diglyceryl ether of polypropylene glycol, approximately 9 repeating units, weight-average molecular weight: approximately 700

• UNIOX HC60, product of NOF Corp.

Polyoxyethylene hydrogenated castor oil, weight-average molecular weight: approximately 3,570

• Vaseline, product of Cognis Japan

Petroleum-derived hydrocarbon, semi-solid
The kinematic viscosities, water holding percentages, weight-average molecular weights, IOBs and melting points of the samples are shown in Table 2.
For the melting point, "<45" indicates a melting point of below 45°C.
Almost the entire skin contact surface of the top sheet of the sanitary napkin was coated with the aforementioned blood slipping agent. Each blood slipping agent was used directly, when the blood slipping agent was liquid at room temperature, or when the blood slipping agent was solid at room temperature it was heated to a temperature of its melting point + 20°C, and then a control seam HMA gun was used for atomization of each blood slipping agent and coating onto the skin contact surface of the top sheet to a basis weight of about 5 g/$m^2$.
Fig. 7 is an electron micrograph of the skin contact surface of a top sheet in a sanitary napkin (No. 1-5) wherein the top sheet comprises tri-C2L oil fatty acid glycerides. As clearly seen in Fig. 7, the tri-C2L oil fatty acid glycerides are present on the fiber surfaces as fine particulates.

[Test methods]

An acrylic board with an opened hole (200 mm × 100 mm, 125 g, with a 40 mm × 10 mm hole opened at the center) was placed on a top sheet comprising each blood slipping agent, and 3.0 g of horse EDTA blood at 37±1°C (obtained by adding ethylenediaminetetraacetic acid (hereunder, "EDTA") to horse blood to prevent coagulation) was dropped through the hole using a pipette (once), and after 1 minute, 3.0 g of horse EDTA blood at 37±1°C was again added dropwise through the acrylic board hole with a pipette (twice).

After the second dropping of blood, the acrylic board was immediately removed and 10 sheets of filter paper (Qualitative filter paper No. 2, product of Advantech Toyo, Inc., 50 mm × 35 mm) (total weight of 10 filter sheets: $FW_0$ (g)) were placed on the location where the blood had been dropped, and then a weight was placed thereover at a pressure of 30 g/cm$^2$. After 1 minute, the filter paper was removed, the total weight $FW_1$ (g) of the 10 tested filter sheets was measured, and the "rewetting rate" was calculated by the following formula.

$$\text{Rewetting rate (mass\%)} = 100 \times [FW_1\ (g) - FW_0\ (g)]/6.0\ (g)$$

In addition to the rewetting rate evaluation, the "absorbent body migration rate" was also measured as the time until migration of blood from the top sheet to the absorbent body after the second dropping of blood. The absorbent body migration rate is the time from introducing the blood onto the top sheet, until the redness of the blood could be seen on the surface and in the interior of the top sheet.

The results for the rewetting rate and absorbent body migration rate are shown below in Table 2.

The whiteness of the skin contact surface of the top sheet (TS) after the absorbent body migration rate test was visually evaluated on the following scale.

VG (Very Good): Virtually no redness of blood remaining, and no clear delineation between areas with and without blood.

G (Good): Slight redness of blood remaining, but difficult to discriminate between areas with and without blood.

F (Fair): Slight redness of blood remaining, areas with blood discernible.

P (Poor): Redness of blood completely remaining.

The tack on the skin contact surface of the top sheet was also measured at 35°C, and evaluated on the following scale.

G: No tack
F: Slight tack
P: Tack

The results are summarized in Table 2 below.

Table 2

| No. | Blood slipping agent | Kinematic viscosity ($mm^2/s$, 40°C) | Water holding percentage (mass%) | Wt.-average mol. wt. | IOB | Melting point (°C) | Rewetting rate (%) | Absorbent body migration rate (sec) | TS whiteness | Tack |
|---|---|---|---|---|---|---|---|---|---|---|
| 1-1 | H-408BRS | 45 | 0.7 | 640 | 0.13 | <-5 | 1.2 | 3 | VG | G |
| 1-2 | H-2408BRS-22 | 22 | 0.8 | 520 | 0.18 | <-5 | 2.0 | 3 | VG | G |
| 1-3 | Cetiol SB45DEO | | | | 0.16 | 44 | 7.0 | 6 | VG | |
| 1-4 | SOY42 | | | 880 | 0.16 | 43 | 5.8 | 8 | VG | G |
| 1-5 | Tri-C2L oil fatty acid glyceride | 20 | <1.0 | 570 | 0.27 | 37 | 0.3 | 3 | VG | G |
| 1-6 | Tri-CL oil fatty acid glyceride | 15 | <1.0 | 570 | 0.28 | 38 | 1.7 | 3 | VG | G |
| 1-7 | PANACET 810s | 9 | 0.3 | 480 | 0.32 | -5 | 2.8 | 3 | VG | G |
| 1-8 | PANACET 800 | 15 | 0.5 | 470 | 0.33 | -5 | 0.3 | 3 | VG | G |
| 1-9 | PANACET 800B | 20 | <1.0 | 470 | 0.33 | -5 | 2.0 | 3 | VG | G |
| 1-10 | NA36 | 40 | <1.0 | 880 | 0.16 | 37 | 3.9 | 5 | VG | G |
| 1-11 | Tri-coconut oil fatty acid glyceride | 25 | <1.0 | 670 | 0.28 | 30 | 4.3 | 5 | VG | G |
| 1-12 | Caprylic acid diglyceride | 25 | 2.7 | 340 | 0.58 | <45 | 4.2 | 9 | G | G |
| 1-13 | UNISTAR H-208BRS | 8 | 0.7 | 360 | 0.24 | <-5 | 2.0 | 5 | VG | G |
| 1-14 | COMPOL BL | 10 | 1.6 | 270 | 0.50 | 2 | 2.0 | 5 | G | G |
| 1-15 | COMPOL BS | 35 | 0.3 | 350 | 0.36 | 37 | 7.9 | 9 | G | G |
| 1-16 | Tributyl O-acetylcitrate | 15 | 0.9 | 400 | 0.60 | <45 | 6.2 | 8 | VG | G |
| 1-17 | Tributyl citrate | 12 | 0.6 | 360 | 0.78 | <45 | 3.0 | 6 | G | G |
| 1-18 | Dioctyl adipate | 7 | 0.4 | 380 | 0.27 | <45 | 1.7 | 6 | VG | G |
| 1-19 | ELECTOL WE20 | 10 | 0.3 | 360 | 0.13 | 29 | 1.8 | 5 | VG | G |
| 1-20 | ELECTOL WE40 | 15 | 0.5 | 390 | 0.12 | 37 | 1.8 | 4 | VG | G |

(continued)

| No. | Blood slipping agent | Kinematic viscosity ($mm^2/s$, 40°C) | Water holding percentage (mass%) | Wt.-average mol. wt. | IOB | Melting point (°C) | Rewetting rate (%) | Absorbent body migration rate (sec) | TS whiteness | Tack |
|---|---|---|---|---|---|---|---|---|---|---|
| 1-21 | UNIOL PB500 | 40 | 3.6 | 500 | 0.44 | <45 | 4.5 | 4 | G | G |
| 1-22 | UNIOL PB700 | 50 | 2.3 | 700 | 0.49 | -5 | 2.8 | 5 | G | G |
| 1-23 | PARLEAM 6 | 5 | 0.06 | 330 | 0.00 | -5 | 6.0 | 8 | VG | G |
| 1-24 | NA50 | 80<< | -* | 880 | 0.18 | 52 | 15.5 | 60 | P | G |
| 1-25 | (Caprylic acid/ Capric acid) monoglyceride | 70 | 4.0<< | 220 | 1.15 | <45 | 4.0 | 4 | P | G |
| 1-26 | 90-L2 Lauric acid monoglyceride | 80<< | 4.0<< | <1,000 | 0.87 | 58 | 6.2 | 7 | P | G |
| 1-27 | Isopropyl citrate | 120 | 4.0<< | 230 | 1.56 | <45 | 12.2 | 5 | G | F |
| 1-28 | Diisostearyl malate | 450 | 4.0<< | 640 | 0.28 | <45 | 5.5 | 8 | F | F |
| 1-29 | UNIOL PB1000R | 70 | 5.5 | 1000 | 0.40 | <45 | 4.0 | 4 | G | F |
| 1-30 | UNIOL D-250 | 20 | 4.0<< | 250 | | <45 | - | - | P | G |
| 1-31 | UNIOL D-400 | 30 | 4.0<< | 400 | 0.76 | <45 | 8.7 | 40 | P | G |
| 1-32 | UNIOL D-700 | 50 | 34.6 | 700 | 0.58 | <45 | 7.5 | - | F | G |
| 1-33 | UNIOL D-1000 | 70 | 26.7 | 1,000 | 0.51 | <45 | 6.8 | 15 | F | F |
| 1-34 | UNIOL D-1200 | 90 | 16.2 | 1,160 | 0.48 | <45 | 0.5 | 11 | F | F |
| 1-35 | UNIOL D-2000 | 160 | | 2,030 | | <45 | - | - | F | P |
| 1-36 | UNIOL D-3000 | | 0.6 | 3,000 | 0.39 | <45 | 1.7 | 10 | F | P |
| 1-37 | UNIOL D-4000 | 450 | 0.5 | 4,000 | 0.38 | <45 | 1.0 | 7 | G | P |
| 1-38 | PEG1500 | 120 | 4.0<< | 1,500-1,600 | 0.78 | 40 | 11.0 | 38 | P | P |
| 1-39 | WILBRITE CP9 | 120 | 0.6 | 1,150 | 0.21 | 35 | 1.4 | 3 | G | P |
| 1-40 | UNILUBE MS-70K | 50 | 2.8 | 1,140 | 0.30 | <-10 | 6.7 | 3 | G | F |
| 1-41 | NONION S-6 | 65 | 4.0<< | 880 | 0.44 | 37 | 8.4 | 7 | P | G |

(continued)

| No. | Blood slipping agent | Kinematic viscosity ($mm^2/s$, 40°C) | Water holding percentage (mass%) | Wt.-average mol. wt. | IOB | Melting point (°C) | Rewetting rate (%) | Absorbent body migration rate (sec) | TS whiteness | Tack |
|---|---|---|---|---|---|---|---|---|---|---|
| 1-42 | UNILUBE 5TP-300KB | 310 | 3.9 | 4,130 | 0.39 | <45 | 2.0 | 6 | G | P |
| 1-43 | WILBRITE s753 | 120 | 27.3 | 960 | 0.67 | -5 | 9.3 | 9 | F | F |
| 1-44 | UNIOL TG-330 | 30 | | 330 | 1.27 | <45 | - | - | - | G |
| 1-45 | UNIOL TG-1000 | 100 | 21.2 | 1,000 | 0.61 | <45 | 14.2 | 7 | G | G |
| 1-46 | UNIOL TG-3000 | 230 | 4.3 | 3,000 | 0.42 | <45 | 0.8 | 6 | G | P |
| 1-47 | UNIOL TG-4000 | 300 | 2.4 | 4,000 | 0.40 | <45 | 2.0 | 6 | G | P |
| 1-48 | UNILUBE DGP-700 | 200 | 4.0<< | 700 | 0.91 | <0 | 8.0 | 10 | F | F |
| 1-49 | UNIOX HC60 | 1150 | | 3,570 | 0.46 | 33 | 14.6 | 46 | P | P |
| 1-50 | Vaseline | 80<< | 0.0 | <1,000 | 0.00 | 55 | 9.7 | 10 | F | P |
| 1-51 | None | - | - | - | - | - | 22.7 | 60< | P | G |

*High viscosity, unmeasurable.

In the absence of a blood slipping agent, the rewetting rate was 22.7% and the absorbent body migration rate was greater than 60 seconds, but the glycerin and fatty acid triesters all produced rewetting rates of 7.0% or less and absorbent body migration rates of no longer than 8 seconds, and therefore significantly improved the absorption performance.
Since the blood slipping agent has a high absorbent body migration rate, this suggests that in the nonwoven fabric of this disclosure, the higher basis weight of the blood slipping agent in the blood slipping agent-containing second region and/or the blood slipping agent-containing fourth region than the basis weight of the blood slipping agent in the blood slipping agent-containing third region causes menstrual blood that has reached the second region and/or fourth region to rapidly slip into the absorbent body before being diffused in the longwise direction.
Similarly, it was found that the absorption performance is greatly improved with a blood slipping agent having a kinematic viscosity of about 0.01 to 80 $mm^2/s$ at 40°C, a water holding percentage of about 0.01 to about 4.0 mass%, and a weight-average molecular weight of less than about 1,000.
Next, several volunteer participants were asked to wear sanitary napkins Nos. 1-1 to 1-51, and the obtained responses indicated that with the sanitary napkins comprising blood slipping agent Nos. 1-1 to 1-23, the top sheets had no sticky feel and the top sheets were smooth, even after absorption of menstrual blood.
Also, with sanitary napkins that comprised blood slipping agent Nos. 1-11, 1-13, 1-16, 1-18 to 1-20 and 1-23, the skin contact surfaces of the top sheets after absorption of menstrual blood was not reddened by the blood and the unpleasantness was minimal.

[Example 2]

[Surface residue rate of menstrual blood on top sheet with ridge-furrow structure]

The surface residue rate of menstrual blood on a top sheet with a ridge-furrow structure was evaluated.
There were prepared a top sheet, formed of a hydrophilic agent-treated air-through nonwoven fabric (composite fiber composed of polyester and polyethylene terephthalate, basis weight: 35 $g/m^2$), a second sheet, formed of an air-through nonwoven fabric (composite fiber composed of polyester and polyethylene terephthalate, basis weight: 30 $g/m^2$), an absorbent body comprising pulp (basis weight: 150 to 450 $g/m^2$, increased at the center section), an acrylic super-absorbent polymer (basis weight: 15 $g/m^2$) and tissue as a core wrap, a water-repellent agent-treated side sheet, and a back sheet composed of a polyethylene film.
The top sheet was a top sheet produced by the method described in Japanese Unexamined Patent Publication No. 2008-2034, having a ridge-furrow structure, with a ridge thickness of approximately 1.5 mm and a furrow thickness of approximately 0.4 mm, the pitch of the ridge-furrow structure (ridge width + furrow width) was approximately 4 mm, and open holes were formed in the furrows at an open area of approximately 15%.
UNISTAR H-408BRS (product of NOF Corp., tetraester of pentaerythritol and fatty acid) was selected as the blood slipping agent, and it was coated onto the skin contact surface (ridge-furrow side) of the top sheet from a control seam HMA gun at room temperature, to a basis weight of 5.0 $g/m^2$. With an electron microscope it was confirmed that the H-408BRS was adhering onto the fiber surfaces as fine particulates.
A back sheet, an absorbent body, a second sheet, and a top sheet with the ridge-furrow side facing upward, were stacked in that order to form sanitary napkin No.2-1.
Sanitary napkins No.2-2 to No.2-40 were produced, changing the blood slipping agent from UNISTAR H-408BRS to the ones listed in Table 3. Each blood slipping agent was used directly, when it was liquid at room temperature, or when the blood slipping agent was solid at room temperature it was heated to its melting point of +20°C, and then a control seam HMA gun was used for atomization of the blood slipping agent and coating onto the skin contact surface of the top sheet to a basis weight of about 5 $g/m^2$.
The blood slipping agent was coated onto essentially the entire skin contact surface of the top sheet, and on both the ridges and furrows.

[Test methods]

After measuring the weight: $W_2$ (g) of the top sheet (the weight of the top sheet before the test), an acrylic board with an opened hole (200 mm × 100 mm, 125 g, with a 40 mm × 10 mm hole opened at the center) was placed on the top sheet, at the center section in the lengthwise direction and widthwise direction of the absorbent article, and 4.0 g of horse EDTA blood at 37±1°C (obtained by adding ethylenediaminetetraacetic acid (hereunder, "EDTA") to horse blood to prevent coagulation) was dropped through the hole using a pipette.
After dropping the horse EDTA blood, the acrylic board was immediately removed, the top sheet was taken off, the mass $W_3$ (g) (mass of the top sheet after the test) was measured and the "surface residue rate A (mass%)" was calculated by the following formula.

$$\text{Surface residue rate (mass\%)} = 100 \times [W_3\ (g) - W_2\ (g)]/4.0\ (g)$$

The results are shown in Table 3 below.

Table 3

| No. | Blood slipping agent | Surface residue rate (mass%) |
|---|---|---|
| 2-1 | H-408BRS | 0.8 |
| 2-2 | H-2408BRS-22 | 0.8 |
| 2-3 | PANACET 810s | 0.8 |
| 2-4 | PANACET 800 | 1.8 |
| 2-5 | Caprylic acid diglyceride | 1.0 |
| 2-6 | UNISTAR H-208BRS | 0.5 |
| 2-7 | COMPOL BL | 1.3 |
| 2-8 | COMPOL BS | 2.5 |
| 2-9 | Tributyl O-acetylcitrate | 0.5 |
| 2-10 | Tributyl citrate | 1.8 |
| 2-11 | Dioctyl adipate | 1.5 |
| 2-12 | ELECTOL WE20 | 0.5 |
| 2-13 | ELECTOL WE40 | 2.3 |
| 2-14 | UNIOL PB500 | 2.5 |
| 2-15 | UNIOL PB700 | 1.3 |
| 2-16 | PARLEAM 6 | 2.0 |
| 2-17 | NA50 | 4.3 |
| 2-18 | (Caprylic acid/Capric acid) monoglyceride | 5.0 |
| 2-19 | 90-L2 Lauric acid monoglyceride | 5.0 |
| 2-20 | Isopropyl citrate | 4.8 |
| 2-21 | Diisostearyl malate | 3.3 |
| 2-22 | UNIOL PB1000R | 2.5 |
| 2-23 | UNIOL D-250 | 3.8 |
| 2-24 | UNIOL D-400 | 4.8 |
| 2-25 | UNIOL D-700 | 4.8 |
| 2-26 | UNIOL D-1000 | 3.8 |
| 2-27 | UNIOL D-1200 | 3.0 |
| 2-28 | UNIOL D-3000 | 3.0 |
| 2-29 | UNIOL D-4000 | 2.5 |
| 2-30 | PEG1500 | 5.5 |
| 2-31 | WILBRITE CP9 | 6.8 |
| 2-32 | UNILUBE MS-70K | 1.5 |
| 2-33 | UNILUBE 5TP-300KB | 2.0 |
| 2-34 | WILBRITE s753 | 3.5 |

(continued)

| No. | Blood slipping agent | Surface residue rate (mass%) |
|---|---|---|
| 2-35 | UNIOL TG-1000 | 3.5 |
| 2-36 | UNIOL TG-3000 | 1.0 |
| 2-37 | UNIOL TG-4000 | 2.0 |
| 2-38 | UNILUBE DGP-700 | 3.5 |
| 2-39 | Vaseline | 4.0 |
| 2-40 | None | 7.5 |

With sanitary napkin No. 2-40, which had no blood slipping agent, the surface residue rate was 7.5 mass%, but with sanitary napkins No. 2-1 to No. 2-16 wherein the kinematic viscosity and water holding percentage were within the prescribed ranges, the surface residue rate was 2.5 mass% or lower.

With sanitary napkins No. 2-1 to No. 2-16, it was observed that the horse EDTA blood that was dropped onto the ridges of the top sheet slipped down from the ridges into the furrows, and was rapidly absorbed from the furrows into the absorbent body. However, with sanitary napkin No. 2-40 which had no blood slipping agent, the dropped horse EDTA blood did not slip down into the furrows but slowly dripped down into the furrows, most of it remaining on the ridges of the top sheet. Also, with the absorbent articles with high water holding percentage, as with No. 2-25, for example, the horse EDTA blood that was dropped onto the ridges of the top sheet did not slip down into the furrows but slowly dripped while partially remaining on the top sheet, and a portion thereof remained on the ridges.

The following experiment was also conducted in order to confirm the function of the blood slipping agent.

[Example 3]

[Viscosity of blood containing blood slipping agent]

The viscosity of the blood slipping agent-containing blood was measured using a Rheometric Expansion System ARES (Rheometric Scientific, Inc.). After adding 2 mass% of PANACET 810s to horse defibrinated blood, the mixture was gently agitated to form a sample, the sample was placed on a 50 mm-diameter parallel plate, with a gap of 100 $\mu$m, and the viscosity was measured at 37$\pm$0.5°C. The sample was not subjected to a uniform shear rate due to the parallel plate, but the average shear rate indicated by the device was 10 $s^{-1}$.

The viscosity of the horse defibrinated blood containing 2 mass% PANACET 810s was 5.9 mPa·s, while the viscosity of the horse defibrinated blood containing no blood slipping agent was 50.4 mPa·s. Thus, the horse defibrinated blood containing 2 mass% PANACET 810s clearly had an approximately 90% lower viscosity than the blood containing no blood slipping agent.

It is known that blood contains components, such as blood cells and has a thixotropic nature, and it is believed that the blood slipping agent of the present disclosure has an effect of lowering the viscosity of blood, such as menstrual blood in the low viscosity range. Lowering the blood viscosity presumably allows absorbed menstrual blood to more easily migrate rapidly from the top sheet to the absorbent body.

[Example 4]

[Photomicrograph of blood slipping agent-containing blood]

Menstrual blood was sampled from healthy volunteers onto thin plastic wrap, and PANACET 810s dispersed in a 10-fold mass of phosphate-buffered saline was added to a portion thereof to a PANACET 810s concentration of 1 mass%. The menstrual blood was dropped onto a slide glass, a cover glass was placed thereover, and the state of the erythrocytes was observed with an optical microscope. A photomicrograph of menstrual blood containing no blood slipping agent is shown in Fig. 8(a), and a photomicrograph of menstrual blood containing PANACET 810s is shown in Fig. 8(b).

From Fig. 8 it is seen that the erythrocytes formed aggregates, such as rouleaux in the menstrual blood containing no blood slipping agent, while the erythrocytes were stably dispersed in the menstrual blood containing PANACET 810s. This suggests that the blood slipping agent functions to stabilize erythrocytes in blood.

[Example 5]

[Surface tension of blood containing blood slipping agent]

The surface tension of blood containing a blood slipping agent was measured by the pendant drop method, using a Drop Master500 contact angle meter by Kyowa Interface Science Co., Ltd. The surface tension was measured after adding a prescribed amount of blood slipping agent to sheep defibrinated blood, and thoroughly shaking.

The measurement was accomplished automatically with a device, and the surface tension $\gamma$ was determined by the following formula (see Fig. 9).

$$\gamma = g \times \rho \times (de)^2 \times 1/H$$

g: Gravitational constant
1/H: Correction factor determined from ds/de
p: Density
de: Maximum diameter
ds: Diameter at location of increase by de from dropping edge

The density $\rho$ was measured at the temperatures listed in Table 4, according to JIS K 2249-1995, "Density test methods and density/mass/volume conversion tables", "5. Vibrating density test method".

The measurement was accomplished using a DA-505 by Kyoto Electronics Co., Ltd.

The results are shown in Table 4 below.

Table 4

| No. | Blood slipping agent | | Measuring temperature (°C) | Surface tension (mN/m) |
|---|---|---|---|---|
| | Type | Amount (mass%) | | |
| 5-1 | - | - | 35 | 62.1 |
| 5-2 | PANACET 810s | 0.01 | 35 | 61.5 |
| 5-3 | | 0.05 | 35 | 58.2 |
| 5-4 | | 0.10 | 35 | 51.2 |
| 5-5 | ELECTOL WE20 | 0.10 | 35 | 58.8 |
| 5-6 | PARLEAM 6 | 0.10 | 35 | 57.5 |
| 5-7 | - | - | 50 | 56.3 |
| 5-8 | WILBRITE cp9 | 0.10 | 50 | 49.1 |

Based on Table 4 it is seen that the blood slipping agent has an effect of lowering the surface tension of blood.

Lowering the surface tension of blood presumably allows absorbed blood to rapidly migrate from the top sheet to the absorbent body, without being retained between the top sheet fibers.

The present disclosure relates to the following J1 to J15.

[J1]

A nonwoven fabric for a top sheet of an absorbent article, which has a longwise direction and a crosswise direction,
wherein the nonwoven fabric has in the crosswise direction a plurality of units each comprising 4 regions: a first region, a second region adjacent to the first region, a third region adjacent to the second region and a fourth region adjacent to the third region, which extend in the longwise direction,
the second region and fourth region have contents of fibers oriented in the longwise direction that are higher than that in the third region,
the first region has a content of fibers oriented in the crosswise direction that is higher than that in the third region,
the second region, third region and fourth region respectively have a blood slipping agent-containing second region, a blood slipping agent-containing third region and a blood slipping agent-containing fourth region containing a blood slipping

agent with a kinematic viscosity of 0.01 to 80 $mm^2/s$ at 40°C, a water holding percentage of 0.01 to 4.0 mass% and a weight-average molecular weight of less than 1,000, and
the basis weight of the blood slipping agent in the blood slipping agent-containing second region and the blood slipping agent-containing fourth region is greater than the basis weight of the blood slipping agent in the blood slipping agent-containing third region.

[J2]

The nonwoven fabric according to J1, wherein the blood slipping agent further has an IOB of 0.00 to 0.60.

[J3]

The nonwoven fabric according to J1 or J2, wherein:
the third region has a content of fibers oriented in the longwise direction of 40% to 80%,
the first region has a content of fibers oriented in the longwise direction of 0% to 45%, and has a content of fibers oriented in the longwise direction that is at least 10% lower than the third region, and
the second region and fourth region have contents of fibers oriented in the longwise direction of 55% or greater, and have contents of fibers oriented in the longwise direction that are at least 10% higher than that in the third region.

[J4]

The nonwoven fabric according to any one of J1 to J3, wherein the first region has a content of fibers oriented in the crosswise direction of 55% or greater.

[J5]

The nonwoven fabric according to any one of J1 to J4, wherein the nonwoven fabric includes a blood slipping agent with a basis weight of 1 to 30 $g/m^2$ in the blood slipping agent-containing second region and the blood slipping agent-containing fourth region.

[J6]

The nonwoven fabric according to any one of J1 to J5, wherein the nonwoven fabric has, in the blood slipping agent-containing third region, a basis weight of blood slipping agent that is 1 to 70 mass% of the basis weight of the blood slipping agent in the blood slipping agent-containing second region and blood slipping agent-containing fourth region.

[J7]

The A nonwoven fabric according to any one of J1 to J6, wherein the nonwoven fabric has a plurality of ridges and a plurality of furrows extending in the longwise direction, the first regions constitute the furrows, the second regions and fourth regions respectively constitute the sides of the ridges, and the third regions constitute the center sections of the ridges.

[J8]

The nonwoven fabric according to any one of J1 to J7, wherein the blood slipping agent is selected from the group consisting of following items (i) to (iii), and any combination thereof:

(i) a hydrocarbon;
(ii) a compound having (ii-1) a hydrocarbon moiety and (ii-2) one or more, same or different groups selected from the group consisting of carbonyl group (-CO-) and oxy group (-O-) inserted between a C-C single bond of the hydrocarbon moiety; and
(iii) a compound having (iii-1) a hydrocarbon moiety, (iii-2) one or more, same or different groups selected from the group consisting of carbonyl group (-CO-) and oxy group (-O-), inserted between a C-C single bond of the hydrocarbon moiety, and (iii-3) one or more, same or different groups selected from the group consisting of carboxyl group (-COOH) and hydroxyl group (-OH), substituting a hydrogen on the hydrocarbon moiety;

with the proviso that when two or more oxy groups are inserted in the compound of (ii) or (iii), the oxy groups are not adjacent.

[J9]

The nonwoven fabric according to any one of J1 to J8, wherein the blood slipping agent is selected from the group consisting of the following items (i') to (iii'), and any combination thereof:

(i') a hydrocarbon;
(ii') a compound having (ii'-1) a hydrocarbon moiety, and (ii'-2) one or more, same or different bonds selected from the group consisting of carbonyl bond (-CO- ), ester bond (-COO-), carbonate bond (-OCOO-), and ether bond (-O-) inserted between a C-C single bond of the hydrocarbon moiety; and
(iii') a compound having (iii'-1) a hydrocarbon moiety, (iii'-2) one or more, same or different bonds selected from the group consisting of carbonyl bond (-CO- ), ester bond (-COO-), carbonate bond (-OCOO-), and ether bond (-O-) inserted between a C-C single bond of the hydrocarbon moiety, and (iii'-3) one or more, same or different groups selected from the group consisting of carboxyl group (-COOH) and hydroxyl group (-OH) substituting a hydrogen on the hydrocarbon moiety;

with the proviso that when two or more same or different bonds are inserted in the compound of (ii') or (iii'), the bonds are not adjacent.

[J10]

The nonwoven fabric according to any one of J1 to J9, wherein the blood slipping agent is selected from the group consisting of following items (A) to (F), as well as any combination thereof:

(A) an ester of (A1) a compound having a chain hydrocarbon moiety and 2-4 hydroxyl groups substituting hydrogens on the chain hydrocarbon moiety, and (A2) a compound having a chain hydrocarbon moiety and one carboxyl group substituting a hydrogen on the chain hydrocarbon moiety;
(B) an ether of (B1) a compound having a chain hydrocarbon moiety and 2-4 hydroxyl groups substituting hydrogens on the chain hydrocarbon moiety, and (B2) a compound having a chain hydrocarbon moiety and one hydroxyl group substituting a hydrogen on the chain hydrocarbon moiety;
(C) an ester of (C1) a carboxylic acid, hydroxy acid, alkoxy acid or oxoacid containing a chain hydrocarbon moiety and 2-4 carboxyl groups substituting hydrogens on the chain hydrocarbon moiety, and (C2) a compound having a chain hydrocarbon moiety and one hydroxyl group substituting a hydrogen on the chain hydrocarbon moiety;
(D) a compound having a chain hydrocarbon moiety, and one bond selected from the group consisting of ether bond (-O-), carbonyl bond (-CO-), ester bond (-COO-) and carbonate bond (-OCOO-), inserted between a C-C single bond of the chain hydrocarbon moiety;
(E) a polyoxy $C_3$-$C_6$ alkylene glycol, or alkyl ester or alkyl ether thereof; and
(F) a chain hydrocarbon.

[J11]

The nonwoven fabric according to any one of J1 to J10, wherein the blood slipping agent is selected from the group consisting of ($a_1$) an ester of a chain hydrocarbon tetraol and at least one fatty acid, ($a_2$) an ester of a chain hydrocarbon triol and at least one fatty acid, ($a_3$) an ester of a chain hydrocarbon diol and at least one fatty acid, ($b_1$) an ether of a chain hydrocarbon tetraol and at least one aliphatic monohydric alcohol, ($b_2$) an ether of a chain hydrocarbon triol and at least one aliphatic monohydric alcohol, ($b_3$) an ether of a chain hydrocarbon diol and at least one aliphatic monohydric alcohol, ($c_1$) an ester of a chain hydrocarbon tetracarboxylic acid, hydroxy acid, alkoxy acid or oxoacid with 4 carboxyl groups, and at least one aliphatic monohydric alcohol, ($c_2$) an ester of a chain hydrocarbon tricarboxylic acid, hydroxy acid, alkoxy acid or oxoacid with 3 carboxyl groups, and at least one aliphatic monohydric alcohol, ($c_3$) an ester of a chain hydrocarbon dicarboxylic acid, hydroxy acid, alkoxy acid or oxoacid with 2 carboxyl groups, and at least one aliphatic monohydric alcohol, ($d_1$) an ether of an aliphatic monohydric alcohol and an aliphatic monohydric alcohol, ($d_2$) a dialkyl ketone, ($d_3$) an ester of a fatty acid and an aliphatic monohydric alcohol, ($d_4$) a dialkyl carbonate, ($e_1$) a polyoxy $C_3$-$C_6$ alkylene glycol, ($e_2$) an ester of a polyoxy $C_3$-$C_6$ alkylene glycol and at least one fatty acid, ($e_3$) an ether of a polyoxy $C_3$-$C_6$ alkylene glycol and at least one aliphatic monohydric alcohol, and ($f_1$) a chain alkane, as well as any combination thereof.

[J12]

The nonwoven fabric according to any one of J1 to J11, wherein the blood slipping agent is adhering to surfaces of fibers of the nonwoven fabric.

[J13]

An absorbent article comprising a liquid-permeable top sheet, a liquid-impermeable back sheet and an absorbent body between the top sheet and back sheet, wherein the top sheet is the nonwoven fabric according to any one of J1 to J12.

[J14]

The absorbent article according to J13, wherein the longwise direction is parallel to the lengthwise direction of the absorbent article.

[J15]

The absorbent article according to J13 or J14, which is a sanitary napkin or panty liner.

Reference Signs List

| | |
|---|---|
| 1 | First region |
| 2 | Second region |
| 3 | Third region |
| 4 | Fourth region |
| 5 | Nonwoven fabric |
| 6 | Unit |
| 7 | Ridge |
| 8 | Furrow |
| 12 | Blood slipping agent-containing second region |
| 13 | Blood slipping agent-containing third region |
| 14 | Blood slipping agent-containing fourth region |
| 21 | Absorbent article |
| 22 | Top sheet |
| 23 | Absorbent body |
| 24 | Side flap |
| 25 | Side sheet |
| 26 | Emboss |
| 27 | Blood slipping agent-containing region |
| 28 | Back sheet |
| 31 | Projection |
| 32 | Recess |
| 33 | Skin contact surface |
| 34 | Blood slipping agent |
| 35, 35', 35" | Menstrual blood |

## Claims

**1.** A nonwoven fabric (5) for a top sheet of an absorbent article, which has a longwise direction (L) and a crosswise direction (C),

wherein the nonwoven fabric has in the crosswise direction a plurality of units (6) each comprising four regions: a first region (1), a second region (2) adjacent to the first region, a third region (3) adjacent to the second region and a fourth region (4) adjacent to the third region, which extend in the longwise direction,
the second region (2) and fourth region (4) have contents of fibers oriented in the longwise direction that are

higher than that in the third region (3),
the first region (1) has a content of fibers oriented in the crosswise direction that is higher than that in the third region (3),
the second region (2), third region (3) and fourth region (4) respectively have a blood slipping agent-containing second region (12), a blood slipping agent-containing third region (13) and a blood slipping agent-containing fourth region (14) containing a blood slipping agent with a kinematic viscosity of 0.01 to 80 $mm^2/s$ at 40°C measured according to JIS K 2283:2000, "5. Kinematic Viscosity Test Method", using a Cannon-Fenske reverse-flow viscometer, at a testing temperature of 40°C, a water holding percentage of 0.01 to 4.0 mass% as described in the description and a weight-average molecular weight of less than 1,000 as described in the description, and
the basis weight of the blood slipping agent in the blood slipping agent-containing second region (12) and the blood slipping agent-containing fourth region (14) is greater than the basis weight of the blood slipping agent in the blood slipping agent-containing third region (13).

**2.** The nonwoven fabric according to claim 1, wherein the blood slipping agent further has an IOB of 0.00 to 0.60 as described in the description.

**3.** The nonwoven fabric according to claim 1 or 2, wherein:

the third region (3) has a content of fibers oriented in the longwise direction of 40% to 80%,
the first region (1) has a content of fibers oriented in the longwise direction of 0% to 45%, and has a content of fibers oriented in the longwise direction that is at least 10% lower than the third region (3), and
the second region (2) and fourth region (4) have contents of fibers oriented in the longwise direction of 55% or greater, and have contents of fibers oriented in the longwise direction that are at least 10% higher than that in the third region (3).

**4.** The nonwoven fabric according to any one of claims 1 to 3, wherein the first region (1) has a content of fibers oriented in the crosswise direction of 55% or greater.

**5.** The nonwoven fabric according to any one of claims 1 to 4, wherein the nonwoven fabric includes a blood slipping agent with a basis weight of 1 to 30 $g/m^2$ in the blood slipping agent-containing second region (12) and the blood slipping agent-containing fourth region (14).

**6.** The nonwoven fabric according to any one of claims 1 to 5, wherein the nonwoven fabric has, in the blood slipping agent-containing third region (13), a basis weight of blood slipping agent that is 1 to 70 mass% of the basis weight of the blood slipping agent in the blood slipping agent-containing second region (12) and blood slipping agent-containing fourth region (14).

**7.** The A nonwoven fabric according to any one of claims 1 to 6, wherein the nonwoven fabric has a plurality of ridges (7) and a plurality of furrows (8) extending in the longwise direction, the first regions (1) constitute the furrows (8), the second regions (2) and fourth regions (4) respectively constitute the sides (7’) of the ridges (7), and the third regions (3) constitute the center sections (7") of the ridges (7).

**8.** The nonwoven fabric according to any one of claims 1 to 7, wherein the blood slipping agent is selected from the group consisting of following items (i) to (iii), and any combination thereof:

(i) a hydrocarbon;
(ii) a compound having (ii-1) a hydrocarbon moiety and (ii-2) one or more, same or different groups selected from the group consisting of carbonyl group (-CO-) and oxy group (-O-) inserted between a C-C single bond of the hydrocarbon moiety; and
(iii) a compound having (iii-1) a hydrocarbon moiety, (iii-2) one or more, same or different groups selected from the group consisting of carbonyl group (-CO-) and oxy group (-O-), inserted between a C-C single bond of the hydrocarbon moiety, and (iii-3) one or more, same or different groups selected from the group consisting of carboxyl group (-COOH) and hydroxyl group (-OH), substituting a hydrogen on the hydrocarbon moiety;

with the proviso that when two or more oxy groups are inserted in the compound of (ii) or (iii), the oxy groups are not adjacent.

**9.** The nonwoven fabric according to any one of claims 1 to 8, wherein the blood slipping agent is selected from the

group consisting of the following items (i') to (iii'), and any combination thereof:

(i') a hydrocarbon;
(ii') a compound having (ii'-1) a hydrocarbon moiety, and (ii'-2) one or more, same or different bonds selected from the group consisting of carbonyl bond (-CO- ), ester bond (-COO-), carbonate bond (-OCOO-), and ether bond (-O-) inserted between a C-C single bond of the hydrocarbon moiety; and
(iii') a compound having (iii'-1) a hydrocarbon moiety, (iii'-2) one or more, same or different bonds selected from the group consisting of carbonyl bond (-CO- ), ester bond (-COO-), carbonate bond (-OCOO-), and ether bond (-O-) inserted between a C-C single bond of the hydrocarbon moiety, and (iii'-3) one or more, same or different groups selected from the group consisting of carboxyl group (-COOH) and hydroxyl group (-OH) substituting a hydrogen on the hydrocarbon moiety;

with the proviso that when two or more same or different bonds are inserted in the compound of (ii') or (iii'), the bonds are not adjacent.

**10.** The nonwoven fabric according to any one of claims 1 to 9, wherein the blood slipping agent is selected from the group consisting of following items (A) to (F), as well as any combination thereof:

(A) an ester of (A1) a compound having a chain hydrocarbon moiety and 2-4 hydroxyl groups substituting hydrogens on the chain hydrocarbon moiety, and (A2) a compound having a chain hydrocarbon moiety and one carboxyl group substituting a hydrogen on the chain hydrocarbon moiety;
(B) an ether of (B1) a compound having a chain hydrocarbon moiety and 2-4 hydroxyl groups substituting hydrogens on the chain hydrocarbon moiety, and (B2) a compound having a chain hydrocarbon moiety and one hydroxyl group substituting a hydrogen on the chain hydrocarbon moiety;
(C) an ester of (C1) a carboxylic acid, hydroxy acid, alkoxy acid or oxoacid containing a chain hydrocarbon moiety and 2-4 carboxyl groups substituting hydrogens on the chain hydrocarbon moiety, and (C2) a compound having a chain hydrocarbon moiety and one hydroxyl group substituting a hydrogen on the chain hydrocarbon moiety;
(D) a compound having a chain hydrocarbon moiety, and one bond selected from the group consisting of ether bond (-O-), carbonyl bond (-CO-), ester bond (-COO-) and carbonate bond (-OCOO-), inserted between a C-C single bond of the chain hydrocarbon moiety;
(E) a polyoxy $C_3$-$C_6$ alkylene glycol, or alkyl ester or alkyl ether thereof; and
(F) a chain hydrocarbon.

**11.** The nonwoven fabric according to any one of claims 1 to 10, wherein the blood slipping agent is selected from the group consisting of ($a_1$) an ester of a chain hydrocarbon tetraol and at least one fatty acid, ($a_2$) an ester of a chain hydrocarbon triol and at least one fatty acid, ($a_3$) an ester of a chain hydrocarbon diol and at least one fatty acid, ($b_1$) an ether of a chain hydrocarbon tetraol and at least one aliphatic monohydric alcohol, ($b_2$) an ether of a chain hydrocarbon triol and at least one aliphatic monohydric alcohol, ($b_3$) an ether of a chain hydrocarbon diol and at least one aliphatic monohydric alcohol, ($c_1$) an ester of a chain hydrocarbon tetracarboxylic acid, hydroxy acid, alkoxy acid or oxoacid with 4 carboxyl groups, and at least one aliphatic monohydric alcohol, ($c_2$) an ester of a chain hydrocarbon tricarboxylic acid, hydroxy acid, alkoxy acid or oxoacid with 3 carboxyl groups, and at least one aliphatic monohydric alcohol, ($c_3$) an ester of a chain hydrocarbon dicarboxylic acid, hydroxy acid, alkoxy acid or oxoacid with 2 carboxyl groups, and at least one aliphatic monohydric alcohol, ($d_1$) an ether of an aliphatic monohydric alcohol and an aliphatic monohydric alcohol, ($d_2$) a dialkyl ketone, ($d_3$) an ester of a fatty acid and an aliphatic monohydric alcohol, ($d_4$) a dialkyl carbonate, ($e_1$) a polyoxy $C_3$-$C_6$ alkylene glycol, ($e_2$) an ester of a polyoxy $C_3$-$C_6$ alkylene glycol and at least one fatty acid, ($e_3$) an ether of a polyoxy $C_3$-$C_6$ alkylene glycol and at least one aliphatic monohydric alcohol, and ($f_1$) a chain alkane, as well as any combination thereof.

**12.** The nonwoven fabric according to any one of claims 1 to 11, wherein the blood slipping agent is adhering to surfaces of fibers of the nonwoven fabric.

**13.** An absorbent article (21) for absorbing blood comprising a liquid-permeable top sheet (22), a liquid-impermeable back sheet (28) and an absorbent body (23) between the top sheet and back sheet, wherein the top sheet (22) is the nonwoven fabric (5) according to any one of claims 1 to 12.

**14.** The absorbent article according to claim 13, wherein the longwise direction is parallel to the lengthwise direction of the absorbent article (21).

15. The absorbent article according to claim 13 or 14, which is a sanitary napkin or panty liner.

**Patentansprüche**

1. Vliesstoff (5) für eine obere Lage eines absorbierenden Artikels, der eine Längsrichtung (L) und eine Querrichtung (C) aufweist,

   wobei der Vliesstoff in der Querrichtung eine Vielzahl von Einheiten (6) aufweist, die jeweils vier Bereiche umfassen: einen ersten Bereich (1), einen zweiten Bereich (2), der dem ersten Bereich benachbart ist, einen dritten Bereich (3), der dem zweiten Bereich benachbart ist, und einen vierten Bereich (4), der dem dritten Bereich benachbart ist, die sich in der Längsrichtung erstrecken,
   der zweite Bereich (2) und vierte Bereich (4) einen Gehalt an Fasern, die in der Längsrichtung ausgerichtet sind, aufweisen, der höher ist als der in dem dritten Bereich (3),
   der erste Bereich (1) einen Gehalt an Fasern, die in der Querrichtung ausgerichtet sind, aufweist, der höher ist als der in dem dritten Bereich (3),
   der zweite Bereich (2), dritte Bereich (3) und vierte Bereich (4) einen Blut-Gleitmittel-enthaltenden zweiten Bereich (12), einen Blut-Gleitmittel-enthaltenden dritten Bereich (13) bzw. einen Blut-Gleitmittel-enthaltenden vierten Bereich (14) aufweisen, die ein Blut-Gleitmittel mit Folgendem enthalten: einer kinematischen Viskosität von 0,01 bis 80 $mm^2/s$ bei 40 °C, gemessen gemäß JIS K 2283:2000, "5. Kinematic Viscosity Test Method", unter Verwendung eines Cannon-Fenske-Gegenstrom-Viskosimeters bei einer Testtemperatur von 40 °C, einem Wasseraufnahme-Prozentsatz von 0,01 bis 4,0 Masse-%, wie in der Beschreibung beschrieben, und einem gewichtsmittleren Molekulargewicht von weniger als 1 000, wie in der Beschreibung beschrieben, und
   das Flächengewicht des Blut-Gleitmittels in dem Blut-Gleitmittel-enthaltenden zweiten Bereich (12) und dem Blut-Gleitmittel-enthaltenden vierten Bereich (14) größer ist als das Flächengewicht des Blut-Gleitmittels in dem Blut-Gleitmittel-enthaltenden dritten Bereich (13).

2. Vliesstoff nach Anspruch 1, wobei das Blut-Gleitmittel weiter eine IOB von 0,00 bis 0,60 aufweist, wie in der Beschreibung beschrieben.

3. Vliesstoff nach Anspruch 1 oder 2, wobei:

   der dritte Bereich (3) einen Gehalt an Fasern, die in der Längsrichtung ausgerichtet sind, von 40 % bis 80 % aufweist,
   der erste Bereich (1) einen Gehalt an Fasern, die in der Längsrichtung ausgerichtet sind, von 0 % bis 45 % aufweist und einen Gehalt an Fasern, die in der Längsrichtung ausgerichtet sind, aufweist, der mindestens 10 % geringer ist als der dritte Bereich (3), und
   der zweite Bereich (2) und vierte Bereich (4) einen Gehalt an Fasern, die in der Längsrichtung ausgerichtet sind, von 55 % oder mehr aufweisen und einen Gehalt an Fasern, die in der Längsrichtung ausgerichtet sind, aufweisen, der mindestens 10 % höher ist als der in dem dritten Bereich (3).

4. Vliesstoff nach einem der Ansprüche 1 bis 3, wobei der erste Bereich (1) einen Gehalt an Fasern, die in der Querrichtung ausgerichtet sind, von 55 % oder mehr aufweist.

5. Vliesstoff nach einem der Ansprüche 1 bis 4, wobei der Vliesstoff ein Blut-Gleitmittel mit einem Flächengewicht von 1 bis 30 $g/m^2$ in dem Blut-Gleitmittel-enthaltenden zweiten Bereich (12) und dem Blut-Gleitmittel-enthaltenden vierten Bereich (14) einschließt.

6. Vliesstoff nach einem der Ansprüche 1 bis 5, wobei der Vliesstoff in dem Blut-Gleitmittel-enthaltenden dritten Bereich (13) ein Flächengewicht von Blut-Gleitmittel aufweist, das 1 bis 70 Masse-% des Flächengewichts des Blut-Gleitmittels in dem Blut-Gleitmittel-enthaltenden zweiten Bereich (12) und Blut-Gleitmittel-enthaltenden vierten Bereich (14) beträgt.

7. Vliesstoff nach einem der Ansprüche 1 bis 6, wobei der Vliesstoff eine Vielzahl von Rippen (7) und eine Vielzahl von Rillen (8) aufweist, die sich in der Längsrichtung erstrecken, die ersten Bereiche (1) die Rillen (8) bilden, die zweiten Bereiche (2) und vierten Bereiche (4) jeweils die Seiten (7’) der Rippen (7) bilden und die dritten Bereiche (3) die mittleren Abschnitte (7") der Rippen (7) bilden.

8. Vliesstoff nach einem der Ansprüche 1 bis 7, wobei das Blut-Gleitmittel aus der Gruppe, die aus den folgenden Punkten (i) bis (iii) besteht, und jedweder Kombination davon ausgewählt ist:

(i) einem Kohlenwasserstoff;
(ii) einer Verbindung, die Folgendes aufweist: (ii-1) eine Kohlenwasserstoffeinheit und (ii-2) eine oder mehrere, gleiche oder unterschiedliche Gruppen, die aus der Gruppe ausgewählt sind, die aus Carbonylgruppe (-CO-) und Oxygruppe (-O-) besteht, die zwischen eine C-C-Einfachbindung der Kohlenwasserstoffeinheit eingefügt sind; und
(iii) einer Verbindung, die Folgendes aufweist: (iii-1) eine Kohlenwasserstoffeinheit, (iii-2) eine oder mehrere, gleiche oder unterschiedliche Gruppen, die aus der Gruppe ausgewählt sind, die aus Carbonylgruppe (-CO-) und Oxygruppe (-O-) besteht, die zwischen eine C-C-Einfachbindung der Kohlenwasserstoffeinheit eingefügt sind, und (iii-3) eine oder mehrere, gleiche oder unterschiedliche Gruppen, die aus der Gruppe ausgewählt sind, die aus Carboxylgruppe (-COOH) und Hydroxylgruppe (-OH) besteht, die einen Wasserstoff an der Kohlenwasserstoffeinheit substituieren;
mit der Maßgabe, dass bei der Einfügung von zwei oder mehr Oxygruppen in die Verbindung von (ii) oder (iii) die Oxygruppen nicht benachbart sind.

9. Vliesstoff nach einem der Ansprüche 1 bis 8, wobei das Blut-Gleitmittel aus der Gruppe, die aus den folgenden Punkten (i') bis (iii') besteht, und jedweder Kombination davon ausgewählt ist:

(i') einem Kohlenwasserstoff;
(ii') einer Verbindung, die Folgendes aufweist: (ii'-1) eine Kohlenwasserstoffeinheit und (ii'-2) eine oder mehrere, gleiche oder unterschiedliche Bindungen, die aus der Gruppe ausgewählt sind, die aus Folgenden besteht: Carbonylbindung (-CO-), Esterbindung (-COO-), Carbonatbindung (-OCOO-) und Etherbindung (-O-), die zwischen eine C-C-Einfachbindung der Kohlenwasserstoffeinheit eingefügt sind; und
(iii') einer Verbindung, die Folgendes aufweist: (iii'-1) eine Kohlenwasserstoffeinheit, (iii'-2) eine oder mehrere, gleiche oder unterschiedliche Bindungen, die aus der Gruppe ausgewählt sind, die aus Folgenden besteht: Carbonylbindung (-CO-), Esterbindung (-COO-), Carbonatbindung (-OCOO-) und Etherbindung (-O-), die zwischen eine C-C-Einfachbindung der Kohlenwasserstoffeinheit eingefügt sind, und (iii'-3) eine oder mehrere, gleiche oder unterschiedliche Gruppen, die aus der Gruppe ausgewählt sind, die aus Carboxylgruppe (-COOH) und Hydroxylgruppe (-OH) besteht, die einen Wasserstoff an der Kohlenwasserstoffeinheit substituieren;
mit der Maßgabe, dass bei der Einfügung von zwei oder mehr gleichen oder unterschiedlichen Bindungen in die Verbindung von (ii') oder (iii') die Bindungen nicht benachbart sind.

10. Vliesstoff nach einem der Ansprüche 1 bis 9, wobei das Blut-Gleitmittel aus der Gruppe, die aus den folgenden Punkten (A) bis (F) besteht, sowie jedweder Kombination davon ausgewählt ist:

(A) einem Ester von (A1) einer Verbindung, die eine Kettenkohlenwasserstoffeinheit und 2-4 Hydroxylgruppen, die Wasserstoffe an der Kettenkohlenwasserstoffeinheit substituieren, aufweist, und (A2) einer Verbindung, die eine Kettenkohlenwasserstoffeinheit und eine Carboxylgruppe, die einen Wasserstoff an der Kettenkohlenwasserstoffeinheit substituiert, aufweist;
(B) einem Ether von (B1) einer Verbindung, die eine Kettenkohlenwasserstoffeinheit und 2-4 Hydroxylgruppen, die Wasserstoffe an der Kettenkohlenwasserstoffeinheit substituieren, aufweist, und (B2) einer Verbindung, die eine Kettenkohlenwasserstoffeinheit und eine Hydroxylgruppe, die einen Wasserstoff an der Kettenkohlenwasserstoffeinheit substituiert, aufweist;
(C) einem Ester von (C1) einer Carbonsäure, Hydroxysäure, Alkoxysäure oder Oxosäure, die eine Kettenkohlenwasserstoffeinheit und 2-4 Carboxylgruppen, die Wasserstoffe an der Kettenkohlenwasserstoffeinheit substituieren, enthalten, und (C2) einer Verbindung, die eine Kettenkohlenwasserstoffeinheit und eine Hydroxylgruppe, die einen Wasserstoff an der Kettenkohlenwasserstoffeinheit substituiert, aufweist;
(D) einer Verbindung, die Folgendes aufweist: eine Kettenkohlenwasserstoffeinheit und eine Bindung, die aus der Gruppe ausgewählt ist, die aus Etherbindung (-O-), Carbonylbindung (-CO-), Esterbindung (-COO-) und Carbonatbindung (-OCOO-) besteht, die zwischen eine C-C-Einfachbindung der Kettenkohlenwasserstoffeinheit eingefügt sind;
(E) einem Polyoxy-$C_3$-$C_6$-Alkylenglycol oder Alkylester oder Alkylether davon; und
(F) einem Kettenkohlenwasserstoff.

11. Vliesstoff nach einem der Ansprüche 1 bis 10, wobei das Blut-Gleitmittel aus der Gruppe ausgewählt ist, die aus Folgenden besteht: ($a_1$) einem Ester eines Kettenkohlenwasserstofftetraols und mindestens einer Fettsäure, ($a_2$)

einem Ester eines Kettenkohlenwasserstofftriols und mindestens einer Fettsäure, ($a_3$) einem Ester eines Kettenkohlenwasserstoffdiols und mindestens einer Fettsäure, ($b_1$) einem Ether eines Kettenkohlenwasserstofftetraols und mindestens eines aliphatischen einwertigen Alkohols, ($b_2$) einem Ether eines Kettenkohlenwasserstofftriols und mindestens eines aliphatischen einwertigen Alkohols, ($b_3$) einem Ether eines Kettenkohlenwasserstoffdiols und mindestens eines aliphatischen einwertigen Alkohols, ($c_1$) einem Ester einer Kettenkohlenwasserstofftetracarbonsäure, -hydroxysäure, -alkoxysäure oder -oxosäure mit 4 Carboxylgruppen und mindestens eines aliphatischen einwertigen Alkohols, ($c_2$) einem Ester einer Kettenkohlenwasserstofftricarbonsäure, -hydroxysäure, -alkoxysäure oder -oxosäure mit 3 Carboxylgruppen und mindestens eines aliphatischen einwertigen Alkohols, ($c_3$) einem Ester einer Kettenkohlenwasserstoffdicarbonsäure, -hydroxysäure, -alkoxysäure oder -oxosäure mit 2 Carboxylgruppen und mindestens eines aliphatischen einwertigen Alkohols, ($d_1$) einem Ether eines aliphatischen einwertigen Alkohols und eines aliphatischen einwertigen Alkohols, ($d_2$) einem Dialkylketon, ($d_3$) einem Ester einer Fettsäure und eines aliphatischen einwertigen Alkohols, ($d_4$) einem Dialkylcarbonat, ($e_1$) einem Polyoxy-$C_3$-$C_6$-Alkylenglycol, ($e_2$) einem Ester eines Polyoxy-$C_3$-$C_6$-Alkylenglycols und mindestens einer Fettsäure, ($e_3$) einem Ether eines Polyoxy-$C_3$-$C_6$-Alkylenglycols und mindestens eines aliphatischen einwertigen Alkohols und ($f_1$) einem Kettenalkan, sowie jedweder Kombination davon.

**12.** Vliesstoff nach einem der Ansprüche 1 bis 11, wobei das Blut-Gleitmittel an Oberflächen von Fasern des Vliesstoffs haftet.

**13.** Absorbierender Artikel (21) für die Absorbierung von Blut, der Folgendes umfasst: eine flüssigkeitsdurchlässige obere Lage (22), eine flüssigkeitsundurchlässige hintere Lage (28) und einen Saugkörper (23) zwischen der oberen Lage und hinteren Lage, wobei die obere Lage (22) der Vliesstoff (5) nach einem der Ansprüche 1 bis 12 ist.

**14.** Absorbierender Artikel nach Anspruch 13, wobei die Längsrichtung parallel zu der Längsrichtung des absorbierenden Artikels (21) verläuft.

**15.** Absorbierender Artikel nach Anspruch 13 oder 14, der eine Monatsbinde oder Slipeinlage ist.

## Revendications

**1.** Tissu non tissé (5) pour une feuille supérieure d'un article absorbant, qui a une direction longitudinale (L) et une direction transversale (C),

où le tissu non tissé a, dans la direction transversale, une pluralité d'unités (6) comprenant chacune quatre régions : une première région (1), une deuxième région (2) adjacente à la première région, une troisième région (3) adjacente à la deuxième région et une quatrième région (4) adjacente à la troisième région qui se prolongent dans la direction longitudinale,

la deuxième région (2) et la quatrième région (4) présentent une teneur en fibres orientées dans la direction longitudinale qui est plus importante que celle de la troisième région (3),

la première région (1) a une teneur en fibres orientées dans la direction transversale qui est plus importante que celle de la troisième région (3),

la deuxième région (2), la troisième région (3) et la quatrième région (4) comportent respectivement une deuxième région contenant un agent facilitant le glissement du sang (12), une troisième région contenant un agent facilitant le glissement du sang (13) et une quatrième région contenant un agent facilitant le glissement du sang (14) qui contiennent un agent facilitant le glissement du sang ayant une viscosité cinématique, mesurée conformément à la norme JIS K 2283: 2000, « 5. Méthode d'essai de la viscosité cinématique » en utilisant un viscosimètre Canon-Fenske à tube ascendant à une température d'essai de 40 °C, dans la plage de 0,01 à 80 $mm^2/s$ à 40 °C, une capacité de rétention de l'eau dans la plage de 0,01 à 4,0 % en masse, comme décrit dans la description, et une masse moléculaire moyenne en poids inférieure à 1 000, comme décrit dans la description et la masse surfacique de l'agent facilitant le glissement du sang dans la deuxième région contenant l'agent facilitant le glissement du sang (12) et dans la quatrième région contenant l'agent facilitant le glissement du sang (14) est plus élevée que la masse surfacique de l'agent facilitant le glissement du sang dans la troisième région contenant l'agent facilitant le glissement du sang (13).

**2.** Tissu non tissé selon la revendication 1, où l'agent facilitant le glissement du sang a en outre une valeur d'IOB qui va de 0,00 à 0,60, comme décrit dans la description.

3. Tissu non tissé selon la revendication 1 ou 2, où :

la troisième région (3) a une teneur en fibres orientées dans la direction longitudinale qui va de 40 % à 80 %,
la première région (1) a une teneur en fibres orientées dans la direction longitudinale qui va de 0 % à 45 % et a une teneur en fibres orientées dans la direction longitudinale qui est de 10 % plus basse au moins que celle de la troisième région (3) et
la deuxième région (2) et la quatrième région (4) ont une teneur en fibres orientées dans la direction longitudinale égale ou supérieure à 55 % et ont une teneur en fibres orientées dans la direction longitudinale qui est de 10 % plus élevée au moins que celle de la troisième région (3).

4. Tissu non tissé selon l'une quelconque des revendications 1 à 3, où la première région (1) a une teneur en fibres orientées dans la direction transversale qui est égale ou supérieure à 55 %.

5. Tissu non tissé selon l'une quelconque des revendications 1 à 4, où le tissu non tissé inclut un agent facilitant le glissement du sang à une masse surfacique qui va de 1 à 30 g/$m^2$ dans la deuxième région contenant un agent facilitant le glissement du sang (12) et dans la quatrième région contenant un agent facilitant le glissement du sang (14).

6. Tissu non tissé selon l'une quelconque des revendications 1 à 5, où le tissu non tissé a, dans la troisième région contenant un agent facilitant le glissement du sang (13), une masse surfacique d'un agent facilitant le glissement du sang qui correspond à de 1 à 70 % en masse de la masse surfacique d'un agent facilitant le glissement du sang dans la deuxième région contenant un agent facilitant le glissement du sang (12) et dans la quatrième région contenant un agent facilitant le glissement du sang (14).

7. Tissu non tissé selon l'une quelconque des revendications 1 à 6, où le tissu non tissé présente une pluralité d'arêtes (7) et une pluralité de sillons (8) se prolongeant dans la direction longitudinale, les premières régions (1) constituant les sillons (8), les deuxièmes régions (2) et les quatrièmes régions (4) constituant respectivement les côtés (7') des arêtes (7) et les troisièmes régions (3) constituant les parties centrales (7") des arêtes (7).

8. Tissu non tissé selon l'une quelconque des revendications 1 à 7, où l'agent facilitant le glissement du sang est sélectionné dans le groupe consistant en les composés (i) à (iii) suivants et toute combinaison de ceux-ci :

(i) un hydrocarbure ;
(ii) un composé ayant (ii-1) un fragment hydrocarboné et (ii-2) un ou plusieurs groupements, identiques ou différents, sélectionnés dans le groupe consistant en un groupement carbonyle (-CO-) et un groupement oxy (-O-) insérés entre une simple liaison C-C du fragment hydrocarboné ; et
(iii) un composé ayant (iii-1) un fragment hydrocarboné, (iii-2) un ou plusieurs groupements, identiques ou différents, sélectionnés dans le groupe consistant en un groupement carbonyle (-CO-) et un groupement oxy (-O-) insérés entre une simple liaison C-C du fragment hydrocarboné et (iii-3) un ou plusieurs groupements, identiques ou différents, sélectionnés dans le groupe consistant en un groupement carboxyle (-COOH) et un groupement hydroxyle (-OH) substituant un hydrogène sur le fragment hydrocarboné ;

à condition que quand deux ou plusieurs groupements oxys sont insérés dans le composé (ii) ou (iii), les groupements oxys ne sont pas adjacents.

9. Tissu non tissé selon l'une quelconque des revendications 1 à 8, où l'agent facilitant le glissement du sang est sélectionné dans le groupe consistant en les composés (i') à (iii') suivants et toute combinaison de ceux-ci :

(i') un hydrocarbure ;
(ii') un composé ayant (ii'-1) un fragment hydrocarboné et (ii'-2) une ou plusieurs liaisons, identiques ou différentes, sélectionnées dans le groupe consistant en une liaison carbonyle (-CO-), une liaison ester (-COO-), une liaison carbonate (-OCOO-) et une liaison éther (-O-) insérées entre une simple liaison C-C du fragment hydrocarboné ; et
(iii') un composé ayant (iii'-1) un fragment hydrocarboné, (iii'-2) une ou plusieurs liaisons, identiques ou différentes, sélectionnées dans le groupe consistant en une liaison carbonyle (-CO-), une liaison ester (-COO-), une liaison carbonate (-OCOO-) et une liaison éther (-O-) insérées entre une simple liaison C-C du fragment hydrocarbure et (iii'-3) un ou plusieurs groupements, identiques ou différents, sélectionnés dans le groupe consistant en un groupement carboxyle (-COOH) et un groupement hydroxyle (-OH) substituant un hydrogène

sur le fragment hydrocarboné ;

à condition que quand deux ou plusieurs liaisons identiques ou différentes sont insérées dans le composé (ii') ou (iii'), les liaisons ne sont pas adjacentes.

**10.** Tissu non tissé selon l'une quelconque des revendications 1 à 9, où l'agent facilitant le glissement du sang est sélectionné dans le groupe consistant en les composés (A) à (F) suivants, ainsi que toute combinaison de ceux-ci :

(A) un ester (A1) d'un composé ayant un fragment à chaîne hydrocarbonée et 2-4 groupements hydroxyles substituant des hydrogènes sur le fragment à chaîne hydrocarbonée et (A2) d'un composé ayant un fragment à chaîne hydrocarbonée et un groupement carboxyle substituant un hydrogène sur le fragment à chaîne hydrocarbonée ;
(B) un éther (B1) d'un composé ayant un fragment à chaîne hydrocarbonée et 2-4 groupements hydroxyles substituant des hydrogènes sur le fragment à chaîne hydrocarbonée et (B2) d'un composé ayant un fragment à chaîne hydrocarbonée et un groupement hydroxyle substituant un hydrogène sur le fragment à chaîne hydrocarbonée ;
(C) un ester (C1) d'un acide carboxylique, d'un hydroxyacide, d'un alkoxyacide ou d'un oxoacide contenant un fragment à chaîne hydrocarbonée et 2-4 groupements carboxyles substituant des hydrogènes sur le fragment à chaîne hydrocarbonée et (C2) d'un composé ayant un fragment à chaîne hydrocarbonée et un groupement hydroxyle substituant un hydrogène sur le fragment à chaîne hydrocarbonée ;
(D) un composé ayant un fragment à chaîne hydrocarbonée et une liaison sélectionnée dans le groupe consistant une liaison éther (-O-), une liaison carbonyle (-CO-), une liaison ester (-COO-) et une liaison carbonate (-OCOO-) insérée entre une simple liaison C-C du fragment à chaîne hydrocarbonée ;
(E) un poly(oxyalkylène en $C_3$-$C_6$ glycol) ou un ester alkylique ou éther alkylique de celui-ci ; et
(F) une chaîne hydrocarbonée.

**11.** Tissu non tissé selon l'une quelconque des revendications 1 à 10, où l'agent facilitant le glissement du sang est sélectionné dans le groupe consistant en ($a_1$) un ester d'un tétraol à chaîne hydrocarbonée et d'au moins un acide gras, ($a_2$) un ester d'un triol à chaîne hydrocarbonée et d'au moins un acide gras, ($a_3$) un ester d'un diol à chaîne hydrocarbonée et d'au moins un acide gras, ($b_1$) un éther d'un tétraol à chaîne hydrocarbonée et d'au moins un alcool aliphatique monohydrique, ($b_2$) un éther d'un triol à chaîne hydrocarbonée et d'au moins un alcool aliphatique monohydrique, ($b_3$) un éther d'un diol à chaîne hydrocarbonée et d'au moins un alcool aliphatique monohydrique, ($c_1$) un ester d'un acide tétracarboxylique, hydroxyacide, alkoxyacide ou oxoacide à chaîne hydrocarbonée ayant 4 groupements carboxyles et d'au moins un alcool aliphatique monohydrique, ($c_2$) un ester d'un acide tricarboxylique, hydroxyacide, alkoxyacide ou oxoacide à chaîne hydrocarbonée ayant 3 groupements carboxyles et d'au moins un alcool aliphatique monohydrique, ($c_3$) un ester d'un acide dicarboxylique, hydroxyacide, alkoxyacide ou oxoacide à chaîne hydrocarbonée ayant 2 groupements carboxyles et d'au moins un alcool aliphatique monohydrique, ($d_1$) un éther d'un alcool aliphatique monohydrique et d'un alcool aliphatique monohydrique, ($d_2$) une dialkylcétone, ($d_3$) un ester d'un acide gras et d'un alcool aliphatique monohydrique, ($d_4$) un carbonate de dialkyle, ($e_1$) un poly(oxyalkylène en $C_3$-$C_6$ glycol), ($e_2$) un ester d'un poly(oxyalkylène en $C_3$-$C_6$ glycol) et d'au moins un acide gras, ($e_3$) un éther d'un poly(oxyalkylène en $C_3$-$C_6$ glycol) et d'au moins un alcool aliphatique monohydrique et ($f_1$) un alcane à chaîne, et toute combinaison de ceux-ci.

**12.** Tissu non tissé selon l'une quelconque des revendications 1 à 11, où l'agent facilitant le glissement du sang adhère aux surfaces des fibres du tissu non tissé.

**13.** Article absorbant (21) conçu pour absorber le sang qui comprend une feuille supérieure perméable aux liquides (22), une feuille de support imperméable aux liquides (28) et un noyau absorbant (23) entre la feuille supérieure et la feuille de support, où la feuille supérieure (22) est le tissu non tissé (5) selon l'une quelconque des revendications 1 à 12.

**14.** Article absorbant selon la revendication 13, où la direction longitudinale est parallèle à la direction de longueur de l'article absorbant (21).

**15.** Article absorbant selon la revendication 13 ou 14, qui est une serviette périodique ou un protège-slip.

FIG. 1

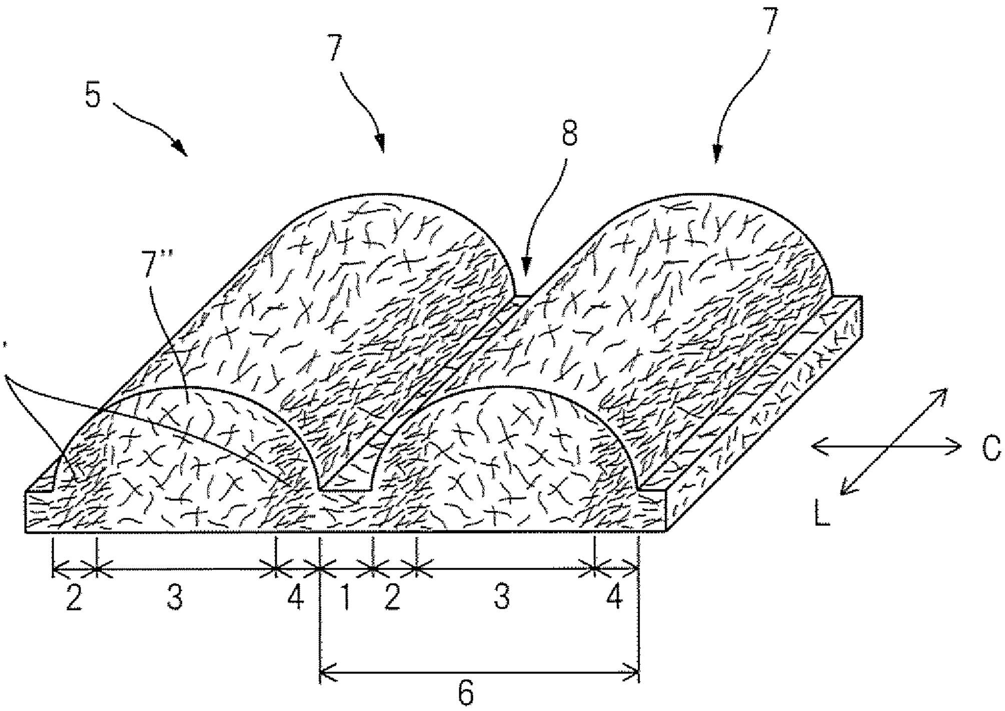


FIG. 2

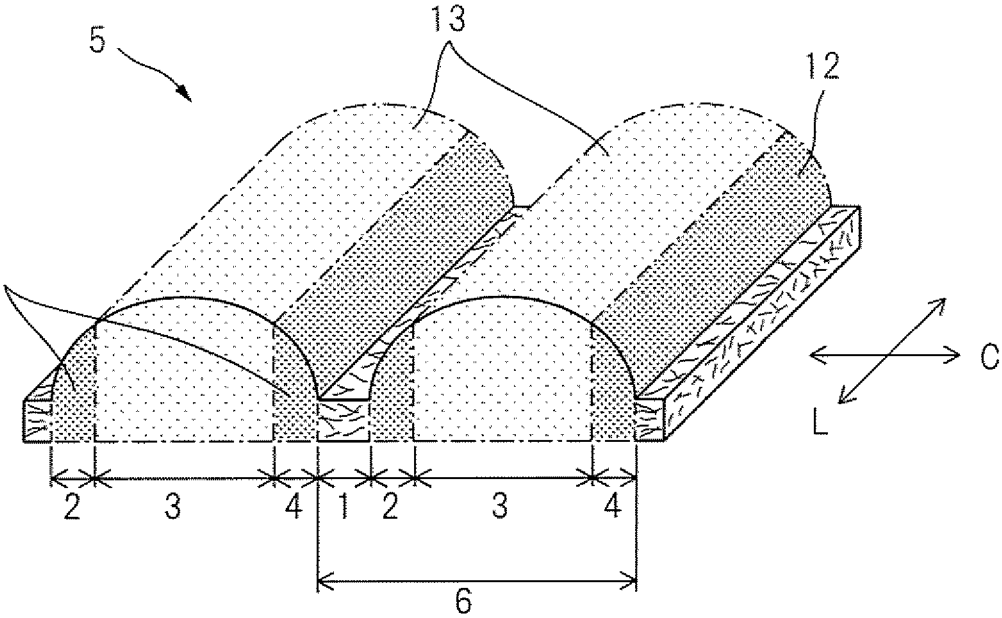

FIG. 3

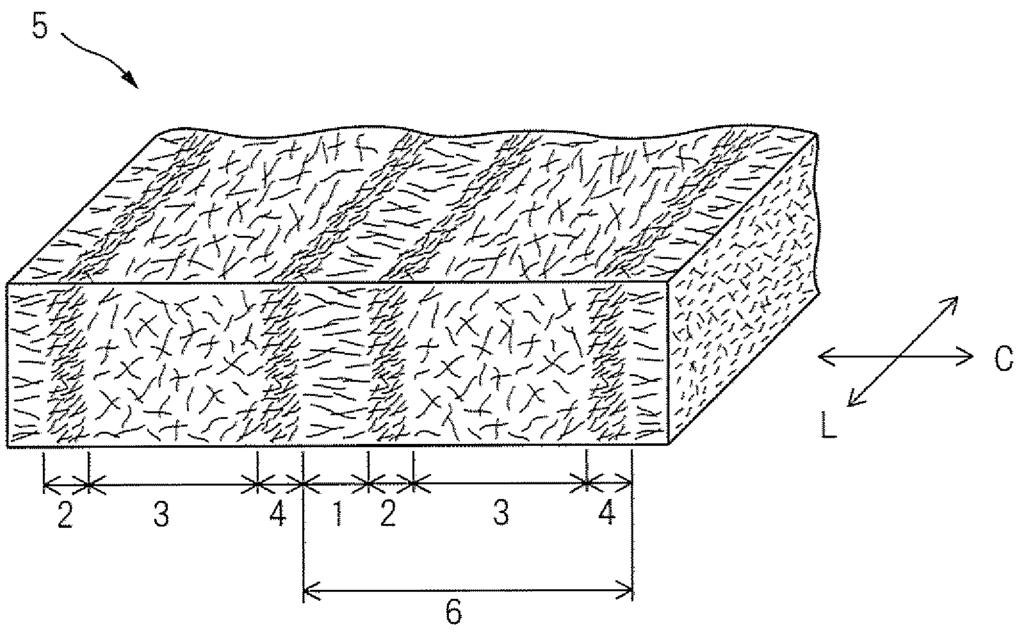


FIG. 4

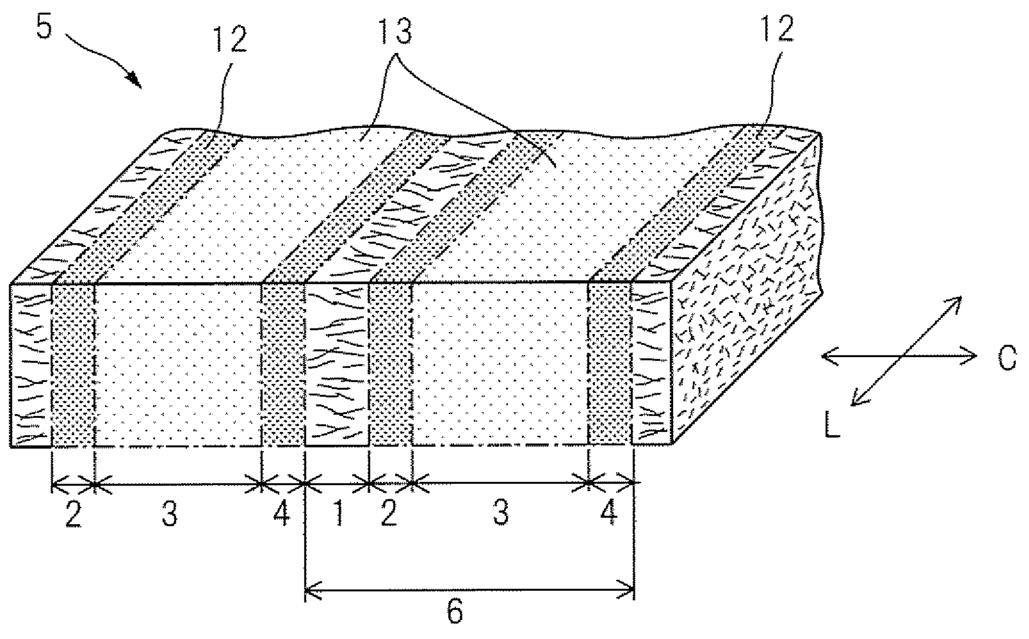

FIG. 5

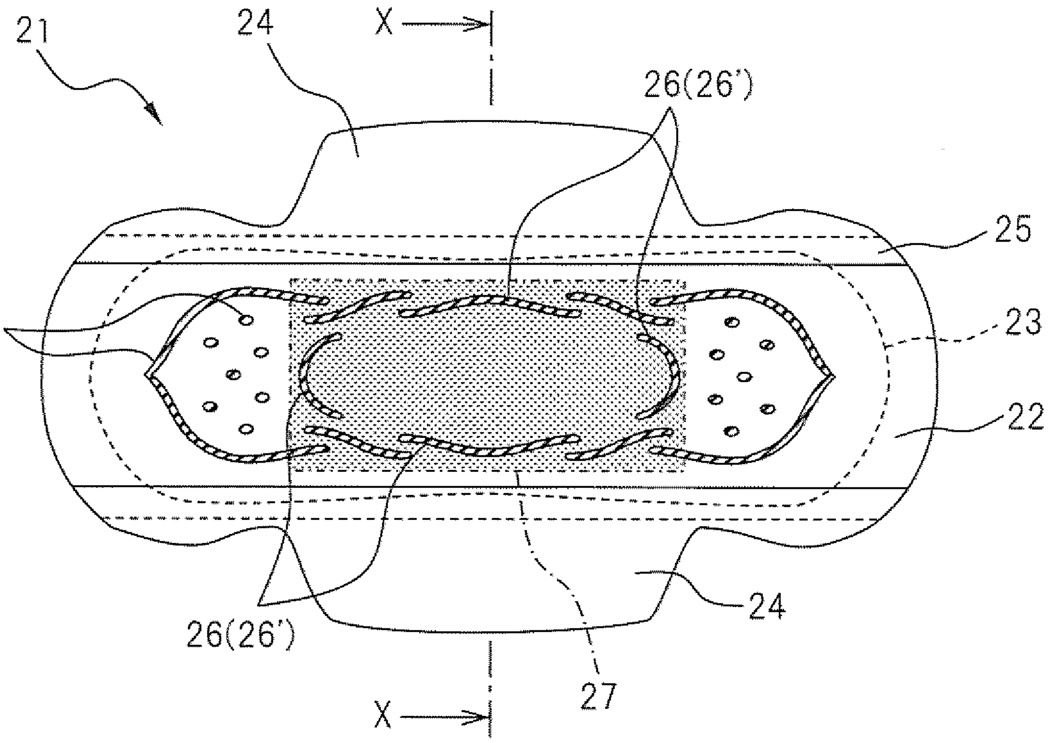


FIG. 6

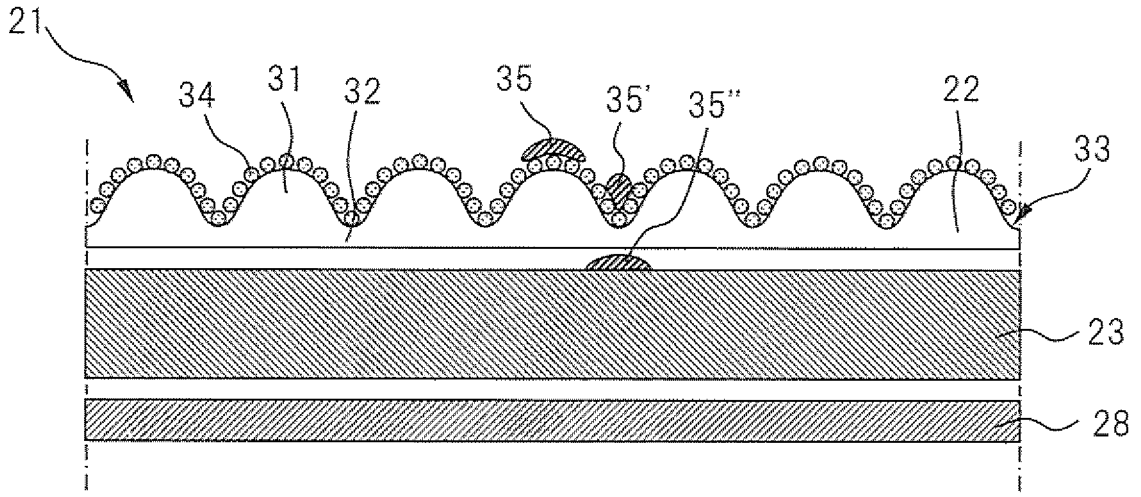

FIG. 7

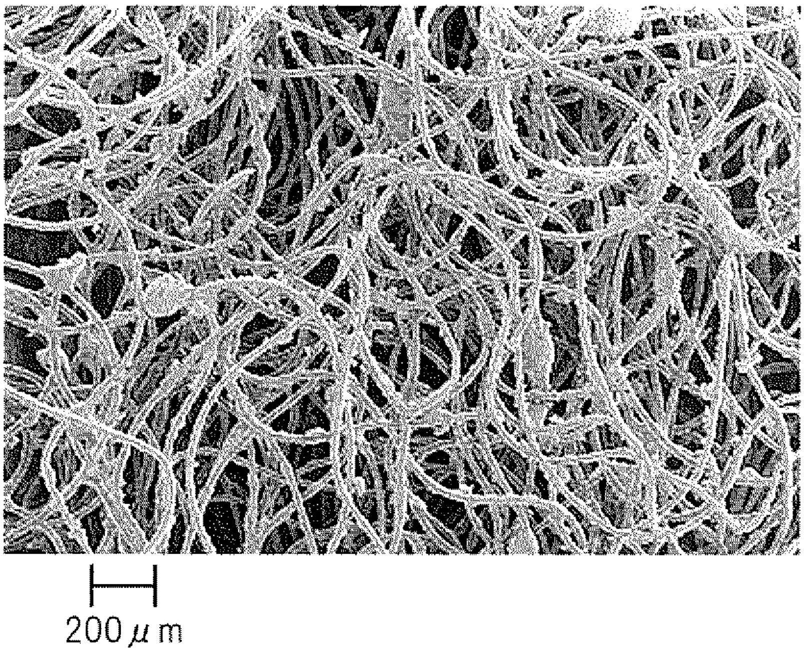

FIG. 8

(a)

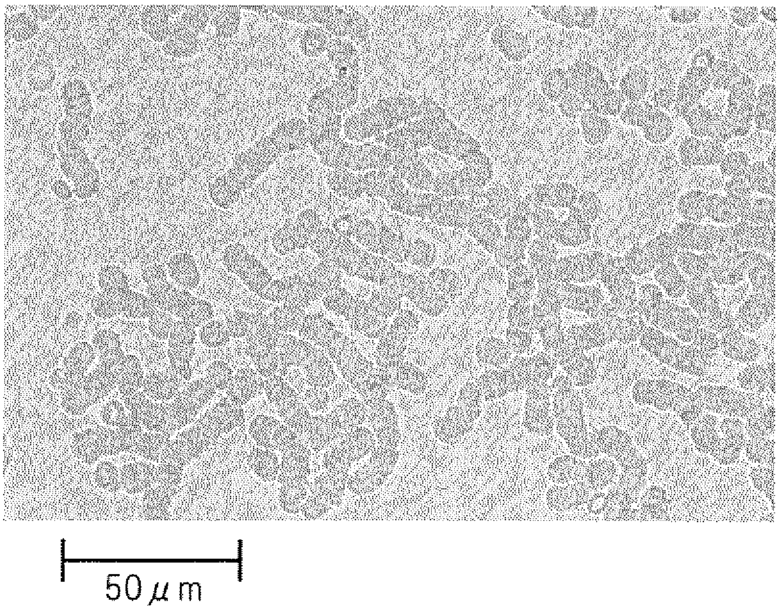


(b)

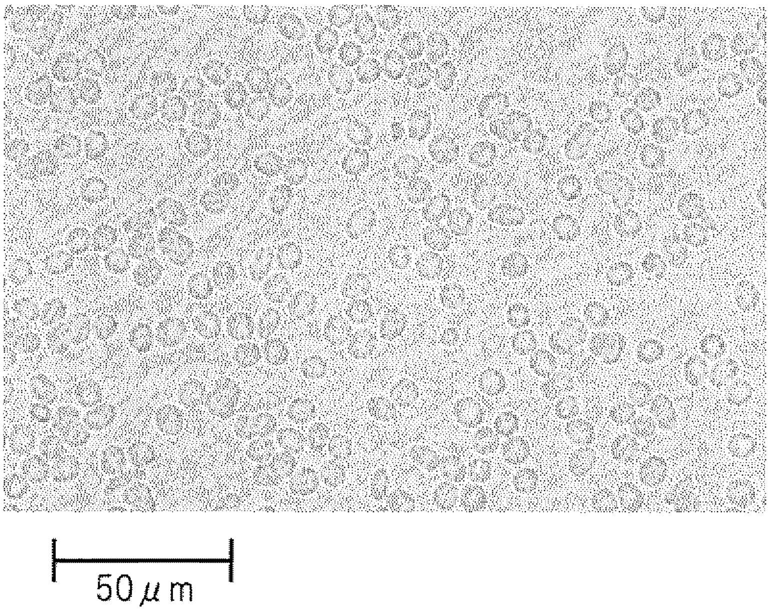

FIG. 9

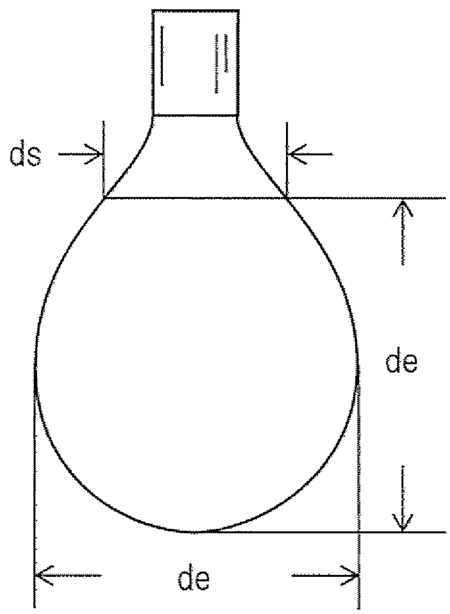

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008025078 A
- JP 2010518918 A
- JP 2011510801 A
- JP 2008002034 A


### Non-patent literature cited in the description


- **FUJITA A. ; KAGAKU NO RYOIKI.** Organic compound predictions and organic paradigms. *Journal of Japanese Chemistry,* 1957, vol. 11 (10), 719-725